# EUROPEAN PATENT SPECIFICATION

(11) **EP 3 958 790 B1**
(45) Date of publication and mention of the grant of the patent: **20.08.2025**
(21) Application number: 20795727.5
(22) Date of filing: 21.04.2020
(51) Int. Cl.: A61F 2/16, A61F 9/007

(54) **AIOL DELIVERY SYSTEM**
AIOL-BEREITSTELLUNGSSYSTEM
SYSTÈME DE MISE EN PLACE D'AIOL

(30) Priority: 22.04.2019 US 201962836956 P; 30.04.2019 US 201962840583 P; 09.12.2019 US 201962945331 P
(43) Date of publication of application: 02.03.2022
(73) Proprietor: Shifamed Holdings, LLC, Campbell, CA 95008 (US)
(72) Inventor: RAQUET, Jacob, Campbell, CA 95008 (US); ARGENTO, Claudio, Campbell, CA 95008 (US); SAUL, Tom, Campbell, CA 95008 (US); PEREA, Juan Diego, Campbell, CA 95008 (US); CROWLEY, Cornelius Matthew, Campbell, CA 95008 (US); BURTON, Cooper, Campbell, CA 95008 (US)
(74) Representative: Mathys & Squire
(86) International application number: PCT/US2020/029131
(87) International publication number: WO 2020/219456

(56) References cited:
- WO-A1-02/074202
- WO-A1-2007/005692
- WO-A1-2009/002789
- WO-A1-2012/129419
- WO-A1-2017/221196
- WO-A1-2017/221196
- WO-A1-99/59668
- US-A1- 2008 027 461
- US-A1- 2015 088 149
- US-A1- 2016 256 316
- US-A1- 2018 177 589

## Description

### CROSS-REFERENCE TO RELATED APPLICATION(S)

This application claims priority to U.S. Provisional Patent Application No. 62/836,956, titled AIOL DELIVERY DEVICE, filed April 22, 2019; U.S. Provisional Patent Application No. 62/840,583, titled AIOL DELIVERY DEVICE, filed April 30, 2019; and U.S. Provisional Patent Application No. 62/945,331, titled AIOL DELIVERY DEVICE, filed December 9, 2019.

### TECHNICAL FIELD

The present technology generally relates to implantable medical devices and, in various aspects, to systems and associated methods for delivering an accommodating intraocular lens (hereinafter "AIOL") or intraocular lens (hereinafter "IOL").

### BACKGROUND

Cataracts affect a large percentage of the worldwide adult population and can cause clouding of the native crystalline lens and, in some cases, vision loss. Patients with cataracts can be treated by native lens removal and surgical implantation of a synthetic IOL. In the United States, 3.5 million IOL implantation procedures are performed annually, while worldwide over 20 million IOL implantation procedures are performed annually.

Although IOL implantation procedures can be effective at restoring vision, conventional IOLs have several drawbacks. For example, many conventional IOLs are not able to change focus as a natural lens would (known as accommodation). Conventional IOLs may be subject to refractive errors that occur after implantation and may require glasses for correcting distance vision. Additionally, in other cases conventional IOLs can be effective in providing far vision but patients may still need glasses for intermediate and near vision.

AIOLs have been proposed to provide accommodative optical power in response to the distance at which a patient views an object. However, devices and systems for delivering such AIOLs are generally still in development and have different drawbacks. For example, conventional delivery systems may require the incision in the eye to be larger than desired for patient recovery. Additionally, conventional delivery systems may not be capable of reliably delivering the AIOL into the eye in the intended configuration.

WO 2017/221196 describes an intraocular lens (IOL) insertion apparatus that may include a hand piece body having a distal tip, an IOL folding chamber, and IOL a dwell position, and a telescoping plunger having a first plunger portion and a second plunger portion. The first plunger portion and second plunger portion may be arranged to simultaneously advance through a first portion of a displacement of the telescoping plunger, and one of the first and second plunger portions may be arranged to advance through a second portion of the displacement of the telescoping plunger.

The present invention is set out in the appended claims.

### BRIEF DESCRIPTION OF THE DRAWINGS

Many aspects of the present technology can be better understood with reference to the following drawings. The components in the drawings are not necessarily drawn to scale. Instead, emphasis is placed on illustrating clearly the principles of the present technology. Furthermore, components can be shown as transparent in certain views for clarity of illustration only and not to indicate that the component is necessarily transparent. Components may also be shown schematically.
FIG. 1 illustrates an AIOL delivery system configured in accordance with an embodiment of the present technology.
FIG. 2 is an exploded view of the delivery system of FIG. 1.
FIG. 3 is a cross-sectional view of the delivery system of FIG. 2.
FIG. 4 illustrates a piston configured in accordance with an embodiment of the present technology.
FIG. 5 illustrates a piston configured in accordance with another embodiment of the present technology.
FIG. 6 illustrates a piston configured in accordance with a further embodiment of the present technology.
FIG. 7 illustrates a funneling insert with a beveled distal tip portion configured in accordance with an embodiment of the present technology.
FIG. 8 illustrates a funneling insert with a constriction configured in accordance with an embodiment of the present technology.
FIG. 9 illustrates a funneling insert with a constriction configured in accordance with another embodiment of the present technology.
FIG. 10 illustrates a distal tip portion of a funneling insert inserted through an incision in the eye in accordance with an embodiment of the present technology.
FIG. 11 illustrates a distal tip portion with a constriction that is inserted through an incision in the eye in accordance with an embodiment of the present technology.
FIG. 12A illustrates a distal tip portion with slits inserted through an incision in the eye in accordance with an embodiment of the present technology.
FIG. 12B illustrates delivery of a lens from the distal tip portion of FIG. 12A.
FIG. 13A illustrates a funneling insert in which the distal tip portion comprises a plurality of folds or crimps and is configured in accordance with an embodiment of the present technology.
FIG. 13B is a closeup view of the distal tip portion of the funneling insert of FIG. 13A.
FIG. 14A illustrates a funneling insert in which the distal tip portion comprises a constriction and is configured in accordance with an embodiment of the present technology.
FIG. 14B is a closeup view of the distal tip portion of the funneling insert of FIG. 14A.
FIG. 14C is a closeup view of a distal tip portion comprising a plurality of flaps connected to each other by a plurality of membrane portions and configured in accordance with an embodiment of the present technology.
FIG. 14D is a closeup view of a distal tip portion comprising a plurality of flaps connected to each other by a membrane and configured in accordance with an embodiment of the present technology.
FIG. 14E is a closeup view of a distal tip portion comprising a plurality of slits and an internal membrane and configured in accordance with an embodiment of the present technology.
FIG. 15A illustrates an AIOL delivery system configured in accordance with an embodiment of the present technology.
FIG. 15B is a side view of the AIOL delivery system of FIG. 15A.
FIG. 15C is a cross-sectional view of the AIOL delivery system of FIG. 15A along line A-A of FIG. 15B.
FIG. 15D illustrates the AIOL delivery system of FIG. 15A in a storage or shipping configuration in accordance with an embodiment of the present technology.
FIG. 15E is a closeup view of an AIOL within the AIOL delivery system of FIG. 15A.
FIG. 16A illustrates an AIOL delivery system configured in accordance with an embodiment of the present technology.
FIG. 16B is a side view of the AIOL delivery system of FIG. 16A.
FIG. 16C is a cross-sectional view of the AIOL delivery system of FIG. 16B along line A-A of FIG. 16B.
FIG. 16D illustrates a cartridge of the AIOL delivery system of FIG. 16A.
FIG. 16E illustrates a side view of the cartridge of FIG. 16D.
FIG. 16F illustrates a cross-sectional view of the cartridge of FIG. 16D along line B-B of FIG. 16E.
FIG. 17A illustrates another AIOL delivery system configured in accordance with an embodiment of the present technology.
FIG. 17B illustrates an injector capable of use with the delivery system of FIG. 17A and configured in accordance with an embodiment of the present technology.
FIG. 17C illustrates the injector of FIG. 17B when disassembled.
FIG. 17D is an exploded view of the tip assembly and injector of the delivery system of FIG. 17A.
FIG. 17E illustrates the tip assembly and injector of FIG. 17D when assembled.
FIG. 17F illustrates the tip assembly and injector of FIG. 17E with the adapter and injector tip omitted for clarity.
FIG. 17G illustrates an injector tip capable of use with the delivery system of FIG. 17A and configured in accordance with an embodiment of the present technology.
FIG. 17H illustrates an elliptical cross-sectional shape capable of use with the injector tip of FIG. 17G in accordance with an embodiment of the present technology.
FIG. 17I illustrates a modified elliptical cross-sectional shape capable of use with the injector tip of FIG. 17G in accordance with an embodiment of the present technology.
FIG. 17J illustrates a rounded hexagonal cross-sectional shape capable of use with the injector tip of FIG. 17G in accordance with an embodiment of the present technology.
FIG. 17K is a perspective view of the plunger assembly of the delivery system of FIG. 17A.
FIG. 17L is an exploded view of the plunger assembly of FIG. 17K.
FIG. 17M is an exploded view of the plunger tip and frame structure of the plunger assembly of FIG. 17L.
FIG. 17N is a top cross-sectional view of the AIOL delivery system of FIG. 17A in an initial configuration in accordance with an embodiment of the present technology.
FIG. 17O is a side cross-sectional view of the AIOL delivery system of FIG. 17N.
FIG. 17P is a top cross-sectional view of the AIOL delivery system of FIG. 17A during a first stage of AIOL delivery in accordance with an embodiment of the present technology.
FIG. 17Q is a side cross-sectional view of the AIOL delivery system of FIG. 17P.
FIG. 17R is a top cross-sectional view of the AIOL delivery system of FIG. 17A during a second stage of AIOL delivery in accordance with an embodiment of the present technology.
FIG. 17S is a side cross-sectional view of the AIOL delivery system of FIG. 17R.
FIG. 17T is a closeup top cross-sectional view of the AIOL delivery system of FIG. 17R.
FIG. 17U is a closeup side cross-sectional view of the AIOL delivery system of FIG. 17R.
FIG. 18A illustrates a plunger tip configured in accordance with an embodiment of the present technology.
FIG. 18B illustrates the plunger tip of FIG. 18A and a frame structure configured in accordance with an embodiment of the present technology.
FIG. 19 illustrates a plunger tip configured in accordance with another embodiment of the present technology.
FIG. 20 illustrates a frame structure configured in accordance with an embodiment of the present technology.
FIG. 21 illustrates a frame structure configured in accordance with another embodiment of the present technology.
FIG. 22 illustrates a plunger tip and frame structure capable of use with a spring-loaded injector and configured in accordance with an embodiment of the present technology.
FIG. 23 illustrates a plunger tip and frame structure with a spring element and configured in accordance with an embodiment of the present technology.
FIG. 24A is an end view of a plunger tip having an end portion with a square or rectangular cross-sectional shape and configured in accordance with an embodiment of the present technology.
FIG. 24B is a top view of the plunger tip of FIG. 24A.
FIG. 24C is a side view of the plunger tip of FIG. 24A.
FIG. 25 is a side view of a plunger tip having a beveled end portion configured in accordance with an embodiment of the present technology.
FIG. 26A is an end view of a plunger tip having an end portion including a pair of protrusions and configured in accordance with an embodiment of the present technology.
FIG. 26B is a top view of the plunger tip of FIG. 26A.
FIG. 26C is a side view of the plunger tip of FIG. 26A.
FIG. 27A is an end view of a plunger tip having a rounded end portion and configured in accordance with an embodiment of the present technology.
FIG. 27B is a top view of the plunger tip of FIG. 27A.
FIG. 27C is a side view of the plunger tip of FIG. 27A
FIG. 28A is a side view of a plunger tip having a sheath structure and configured in accordance with an embodiment of the present technology.
FIG. 28B is an exploded side view of the plunger tip of FIG. 28A.
FIG. 28C is an exploded perspective view of the plunger tip of FIG. 28A.
FIG. 29A is a side view of a plunger tip having a sheath structure with prongs and configured in accordance with an embodiment of the present technology.
FIG. 29B is an exploded side view of the plunger tip of FIG. 29A.
FIG. 29C is an exploded perspective view of the plunger tip of FIG. 29A.

### DETAILED DESCRIPTION

The present technology is generally directed to systems and associated devices and methods for delivering an AIOL. An AIOL delivery system configured in accordance with an embodiment of the present technology can include, for example, a tip assembly configured to couple to an injector. The tip assembly can include an injector tip configured to receive an AIOL. The injector tip can include a distal portion configured to be inserted at least partially into the patient's eye. The tip assembly can also include a plunger assembly positionable at least partially within the injector tip. The plunger assembly can include an inner member configured to engage the AIOL and an outer member positioned at least partially around the outer member. When the plunger assembly is actuated, the inner member can be configured to move distally relative to the outer member to displace the AIOL out of the injector tip and into the patient's eye.

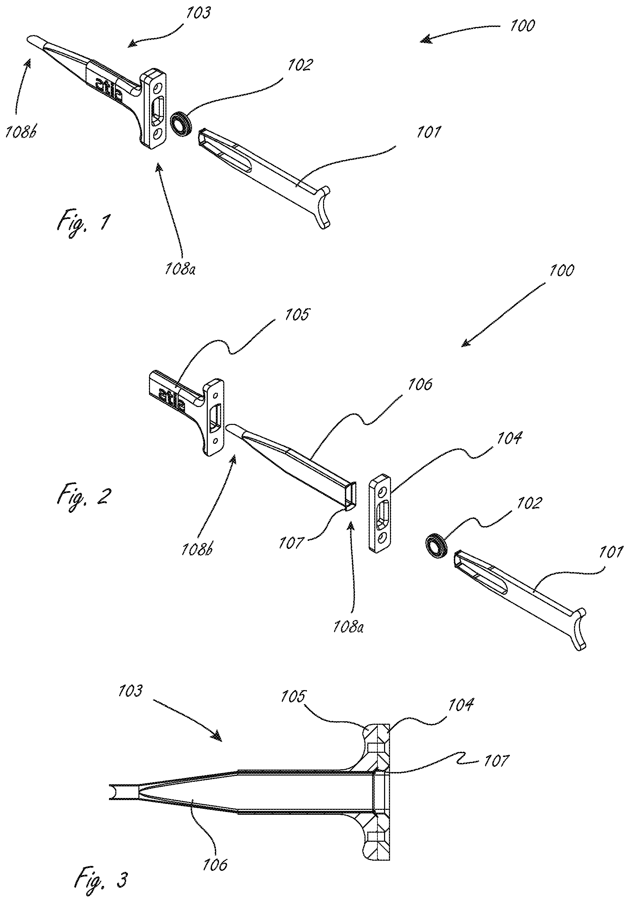

Specific details of various embodiments of the present technology are described below with reference to FIGS. 1-29C. Although certain embodiments are described below with respect to AIOLs and associated methods, other embodiments are within the scope of the present technology. For example, although certain embodiments of delivery systems and devices (e.g., an injector) are described herein in connection with an AIOL, one will appreciate from the description herein that these delivery systems and devices, and various related features and methods, can be used equally with IOLs and other lenses. Additionally, other embodiments of the present technology can have different configurations, components, and/or procedures than those described herein. For instance, AIOL delivery systems configured in accordance with the present technology may include additional elements and features beyond those described herein, or other embodiments may not include several of the elements and features shown and described herein.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

Reference throughout this specification to relative terms such as, for example, "generally," "approximately," and "about" are used herein to mean the stated value plus or minus 10%.

Reference throughout this specification to terms such as "top," "bottom," "lateral," "height," and "width" can refer to relative directions, positions, or dimensions of features in the embodiments herein in view of the orientation shown in the drawings. However, it will be appreciated that the present technology encompasses embodiments having orientations different than the orientation shown in the drawings. For example, in other embodiments a component may be rotated by 90 degrees such that the "height" of the original embodiment corresponds to the "width" in the rotated embodiment, and the "width" of the original embodiment corresponds to the "height" in the rotated embodiment.

The present technology is generally directed to systems and devices for delivering an AIOL into a patient's eye. In some embodiments, for example, an AIOL delivery system includes an injector tip (which may be interchangeably referred to herein as an "injector body," "funnel," or "funneling insert") configured to receive an AIOL therein. The system can further include a plunger (which may be interchangeably referred to herein as a "piston") configured to engage the AIOL to displace the AIOL distally out of the injector tip and into the patient's eye. The components of the system (e.g., the injector tip, plunger, etc.) can be configured to accommodate a relatively small incision in the eye (e.g., an incision less than or equal to 3.5 mm in length) while avoiding or reducing stresses on the AIOL that could rupture or otherwise damage the AIOL. Additionally, the embodiments herein can provide AIOL delivery in a controllable and reliable manner (e.g., without flipping, inverting, or other unwanted movements of the AIOL). Accordingly, the present technology is expected to improve the safety, efficiency, and consistency of AIOL implantation procedure.

In some embodiments, for example, the AIOL delivery systems described herein include a tip assembly configured to deliver an AIOL into a patient's eye. The tip assembly can include an injector tip configured to receive the AIOL. The injector tip can have a tapered shape with a narrower distal portion (e.g., for insertion into the eye) and a wider proximal portion. In some embodiments, for example, the distal portion has a cross-sectional dimension (e.g., diameter, width, height) less than or equal to 3.5 mm, and the proximal portion has a cross-sectional dimension greater than the cross-sectional dimension of the distal portion. The tapered shape of the injector tip can be configured to controllably and consistently deform the AIOL from a resting configuration into a low-profile delivery configuration suitable for introduction into the eye via a relatively small incision. The tip assembly can also include a plunger assembly configured to push the AIOL distally through the injector tip and into the eye. To accommodate the tapered shape of the injector tip, the plunger assembly can have an inner member configured to move telescopically relative to the outer member. As the plunger assembly moves distally through the injector tip, the plunger assembly can transition from a first configuration, in which the inner and outer members push on the AIOL together, to a second configuration, in which only the inner member pushes on the AIOL.

As another example, the AIOL delivery systems described herein can include an injector body configured to receive a piston via an inlet at a proximal portion of the injector body. The inlet can be tapered, flared, and/or otherwise shaped to facilitate direction of the piston into the injector body. The distal portion of the injector body can include an outlet sized and shaped to pass through an incision (e.g., a corneal incision) into the eye. The injector body can include an internal channel that tapers from the inlet to the outlet. The internal channel can be configured to receive an AIOL or other optical device and compress the device between the inlet and the outlet. Optionally, the injector body can include a necked, narrowed, or otherwise constricted portion at or near the distal end of the injector body. Positioning the constricted portion of the injector body proximal to the distal end of the injector body can allow for pre-expansion of the optical device before the optical device exits the outlet of the injector body. In some embodiments, such pre-expansion of the optical device can reduce the risk of injury to the eye during implantation of the optical device.

FIGS. 1-3 illustrate an AIOL delivery system 100 configured in accordance with an embodiment of the present technology. More specifically, FIGS. 1 and 2 are exploded views of the delivery system 100, and FIG. 3 is a cross-sectional view of an injector body 103 of the delivery system 100. Referring first to FIG. 1, the delivery system 100 includes an injector body 103 and a piston 101. The injector body 103 can be configured to receive a lens system or AIOL 102 therein. The AIOL 102 is shown in FIG. 1 positioned proximal of the injector body 103 and distal to the piston 101. The piston 101 can be actuated to push the AIOL 102 out of the outflow portion 108b of the injector body 103 and into a patient's eye.

The injector body 103 can include an inflow (e.g., proximal) portion 108a and an outflow (e.g., distal) portion 108b. The AIOL 102 can be initially placed on, at, or within the inflow portion 108a. When in the inflow portion 108a, the AIOL 102 can be in an undeformed or substantially undeformed configuration. For example, the inflow portion 108a can have a cross-sectional dimension (e.g., area, diameter, width, etc.) configured to accommodate a cross-sectional dimension of the AIOL 102 normal to the optical axis of the AIOL 102 (referred to herein as the cross-section of the AIOL 102) with little or no deformation of the AIOL 102.

The outflow portion 108b can be configured to be at least partially inserted into a patient's eye to deliver the AIOL 102. The outflow portion 108b can have a reduced cross-sectional dimension (e.g., area, diameter, width) compared to the inflow portion 108a. For example, the inflow portion 108a can have a rectangular cross-sectional shape (e.g., a 3 mm x 10 mm rectangular shape), and the outflow portion 108b can have a circular cross-sectional shape (e.g., a 4 mm diameter circular shape). In some embodiments, the cross-sectional dimension of the outflow portion 108b is configured for AIOL delivery through a relatively small corneal incision. For example, the incision can have a length less than or equal to 2.0 mm, 2.5 mm, 3.0 mm, 3.5 mm, 4.0 mm, 4.5 mm, or 5.0 mm. As a result, the AIOL 102 can be delivered from the injector body 103 into the eye, or, optionally, into a delivery tool with a cross-sectional dimension which minimizes or otherwise reduces the length of the incision into the eye, and therefore the size of the injury.

In some embodiments, the outflow (e.g., distal) portion 108b of the injector body 103 is expandable. Optionally, the outflow portion 108b of the injector body 103 is expandable and a portion proximal to the outflow portion 108b is not expandable. The portion proximal the outflow portion 108b (sometimes referred to as an intermediate tip portion) may be more rigid than the outflow portion 108b, for example, to limit pressure on the incision site during delivery. Additional features of the outflow portion 108b are described in greater detail below.

The injector body 103 can be shaped such that the cross-sectional geometry (e.g., size and/or shape) of the inflow portion 108a relatively and monotonically transitions into the cross-sectional geometry of the outflow portion 108b. The AIOL 102 can be pushed through the injector body 103 from the inflow portion 108a to the outflow portion 108b by the piston 101. In some embodiments, the piston 101 includes features (not shown) that allow for the end of the piston 101 in contact with the AIOL 102 to change shape in accordance with the change in shape of the injector body 103. For example, the end of the piston 101 can include one or more weakened portions configured to deflect in response to contact with the injector body 103. This approach is expected to improve the ability of the piston 101 to push on the AIOL 102 from the inflow portion 108a to the outflow portion 108b, as described in greater detail below.

Referring next to FIGS. 2 and 3 together, in some embodiments, the injector body 103 includes a funneling insert 106 that is mounted into a handle or housing 105. The funneling insert 106 can be configured to receive the AIOL 102 therein, and can include the inflow portion 108a and outflow portion 108b. The handle 105 can be used by an operator to hold and grip the delivery system 100 during use. The funneling insert 106 can be received in and secured to the handle 105 by an end cap 104. In the illustrated embodiment, for example, the funneling insert 106 is held in the handle 105 by capturing a flange portion 107 of the funneling insert 106 in an interference fit between the end cap 104 and the handle 105. In other embodiments, however, the injector body 103 can be manufactured as a single unitary component in which the funneling insert 106 and handle 105 are integrally formed with each other.

In some embodiments, at least the surface(s) (e.g., inner surfaces) of the funneling insert 106 are made of, coated with, or otherwise include materials suitable for reducing or minimizing the interfacial friction between the AIOL 102 and the funneling insert 106. In other embodiments the entirety of the funneling insert 106 is made of materials suitable for reducing or minimizing the interfacial friction between the AIOL 102 and the funneling insert 106. In some embodiments, for example, the funneling insert 106 is made of a heat shrink material.

In some embodiments, the funneling insert 106 is manufactured as a single unitary component. In other embodiments, however, the funneling insert 106 can be manufactured as two discrete components, e.g., split along a plane equivalent or parallel to the plane defining the cross-section shown in in FIG. 3. In such embodiments the AIOL 102 can be placed in one half of the funneling insert 106, and then the other half of the funneling insert 106 can be placed and affixed after loading the AIOL 102.

The piston 101 can be configured to push the AIOL 102 through the funneling insert 106. As the piston 101 advances through the funneling insert 106, the leading (e.g., distal) section of the piston 101 can change shape and adapt to the changing cross-section of the funneling insert 106 and proximal surface of the deforming AIOL 102. The proximal end portion of the piston 101 may be sufficiently stiff relative to the AIOL components to deform the AIOL 102 and move it through the funneling insert 106 while the piston sustains reduced or minimal deformation under the applied loads. The proximal end portion of the piston 101 may be imparted with such stiffness by including a stiff material, a rigid insert or support, and/or other mechanisms as would be understood from the description herein. The distal end portion of the piston 101 can include materials and/or features which allow the distal end portion to conform to the cross section of the funneling insert 106. For example, the piston 101 may be made of two or more pieces which are more compliant and/or deformable at regions near the AIOL 102 (e.g., at distal regions of the piston 101). Alternatively, the piston 101 may be made of a single piece having a compliance (material and/or structural) that increases at regions closer to the AIOL 102.

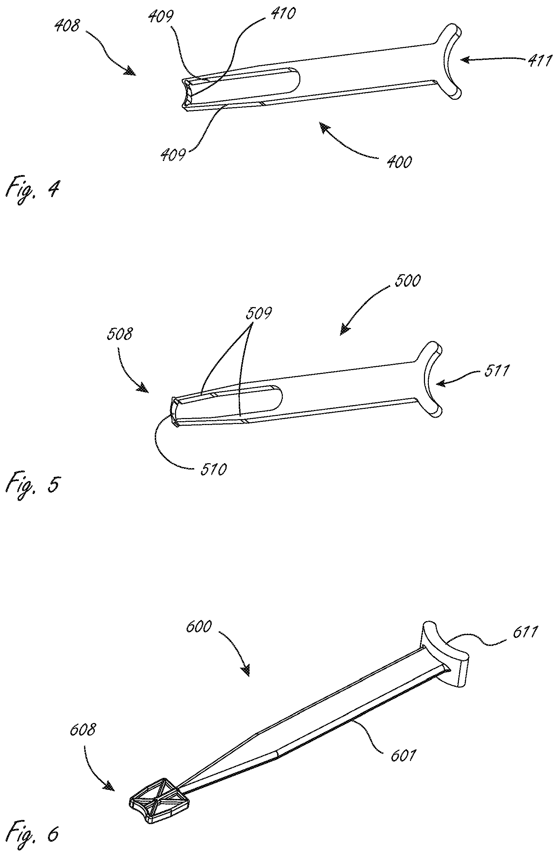

FIGS. 4-6 illustrate various embodiments of pistons capable of use in the AIOL delivery systems described herein. The features of the pistons described with respect to FIGS. 4-6 can combined with each other or with any other embodiment described herein (e.g., piston 101 of FIGS. 1-3).

Referring first to FIG. 4, the piston 400 includes a distal end portion 408 and a proximal end portion 411. The distal end portion 408 can be configured to engage an AIOL (not shown) and the proximal end portion 411 can be configured to be actuated by a user. The piston 400 can be structurally compliant at the distal end portion 408 and stiff on the proximal end portion 411. For example, the distal end portion 408 can include a flexible distal face 410 positioned between two longitudinally-extending arms 409. The arms 409 can be flexible structures that are configured to move towards each other as the piston 400 is advanced through a tapered portion of a funneling insert (e.g., funneling insert 106 of FIG. 1). In the illustrated embodiment, the distal face 410 of the piston 400 has a concave shape that is hinged or otherwise configured to bend and/or deflect in a proximal direction (e.g., toward the proximal end 411) as the arms 409 are pushed together. The proximal end portion 411 can have a curved shape configured to accommodate the user's thumb or other digit during operation of the piston 400.

Referring next to FIG. 5, the piston 500 includes a distal end portion 508 and a proximal end portion 511. The piston 500 can be identical or generally similar to the piston 400 of FIG. 4, except that the distal end portion 508 of the piston 500 has a distal surface 510 with a convex shape that is hinged or otherwise configured to bend and/or deflect in a distal direction as arms 509 are pushed together. In some embodiments, the distal surface 510 has a cross-sectional dimension (e.g., thickness) that decreases monotonically towards the midline of the piston 500.

Referring next to FIG. 6, the piston 600 includes a proximal rigid piston body 601 and a distal deformable piston tip 608. The deformable piston tip 608 can be configured to engage an AIOL (not shown). The deformable piston tip 608 can be any structure having sufficient flexibility and/or elasticity to deform as the piston 600 is advanced through a tapered portion of a funneling insert (e.g., funneling insert 106 of FIG. 1). The deformable piston tip 608 may comprise a foam, a fluid filled bag, a low durometer fluid such as a silicone or polyurethane, or other cohesive viscoelastic material. The piston 600 can include a proximal end portion 611 with an enhanced platform or similar structure that is curved to accommodate a user's thumb or other digit during operation of the piston 600 to implant the AIOL.

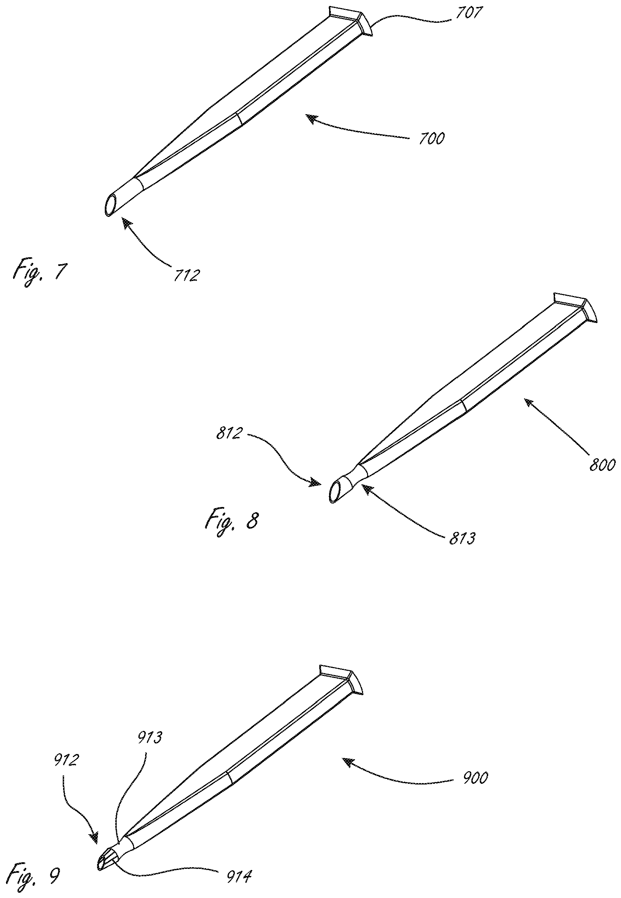

FIGS. 7-14E illustrate various embodiments of funneling inserts capable of use in the AIOL delivery systems described herein. The features of the funneling inserts described with respect to FIGS. 7-14E can be combined with each other or with any other embodiment described herein (e.g., the funneling insert 106 of FIGS. 1-3).

Referring first to FIG. 7, the funneling insert 700 includes a distal tip portion 712 configured to be at least partially inserted into a patient's eye. The distal tip portion 712 can be generally cylindrical with a circular cross-sectional shape. In other embodiments, the distal tip portion 712 can have a different cross-sectional shape (e.g., an oval, square, rectangular, or other cross-sectional shape). In the illustrated embodiment, the distal tip portion 712 includes an angled or beveled cut at its distalmost end. Optionally, the funneling insert 700 can include a proximal flange feature 707. The flange feature 707 can be used to secure the funneling insert 700 to another component (e.g., handle 105 of FIGS. 1-3).

Referring next to FIG. 8, the funneling insert 800 is identical or generally similar to funneling insert 700 of FIG. 7, except that the proximal portion of the distal tip portion 812 comprises a constriction or narrowed portion 813. The constriction 813 can have a reduced cross-section dimension (e.g., area, diameter, width) compared to the rest of the distal tip portion 812. As a result, the constriction 813 can reduce the size of the funneling insert 800 in the region where the funneling insert 800 interfaces with the eye during AIOL injection, which is expected to minimize or reduce injury to the eye while the AIOL is delivered. In some embodiments, as discussed in further detail below, the constriction 813 can allow the AIOL to begin expanding before exiting from the distal tip portion 812. The remaining portions of the distal tip portion 812 (e.g., the portion proximal to the constriction 813) can be expandable or non-expandable.

Referring next to FIG. 9, the funneling insert 900 is identical or generally similar to funneling insert 800 of FIG. 8, except that funneling insert 900 includes a tapered distal tip portion 912. The funneling insert 900 can also include a constriction 913 proximal to the tapered distal tip portion 912. In some embodiments, the tapered distal tip portion 912 comprises one or more slits 914. The slits 914 can be configured such that once the tapered distal tip portion 912 of the funneling insert 900 has been inserted at least partially into the eye via an incision site, the tapered distal tip portion 912 can open up and/or expand while avoiding or reducing the risk of additional injury to the incision site. In this manner, a relatively larger AIOL may be delivered with limited or no enlargement of the incision site. In the illustrated embodiment, the slits 914 are distributed only over the upper portion (e.g., upper half) of the distal tip portion 912. In such embodiments, during use, the slits 914 can be oriented posteriorly in the eye, while the portions of the distal tip portion 912 without slits can be oriented anteriorly in the eye and can be used to direct the AIOL into the eye capsule. In other embodiments, however, the slits 914 can be distributed around the entire circumference of the distal tip portion 912.

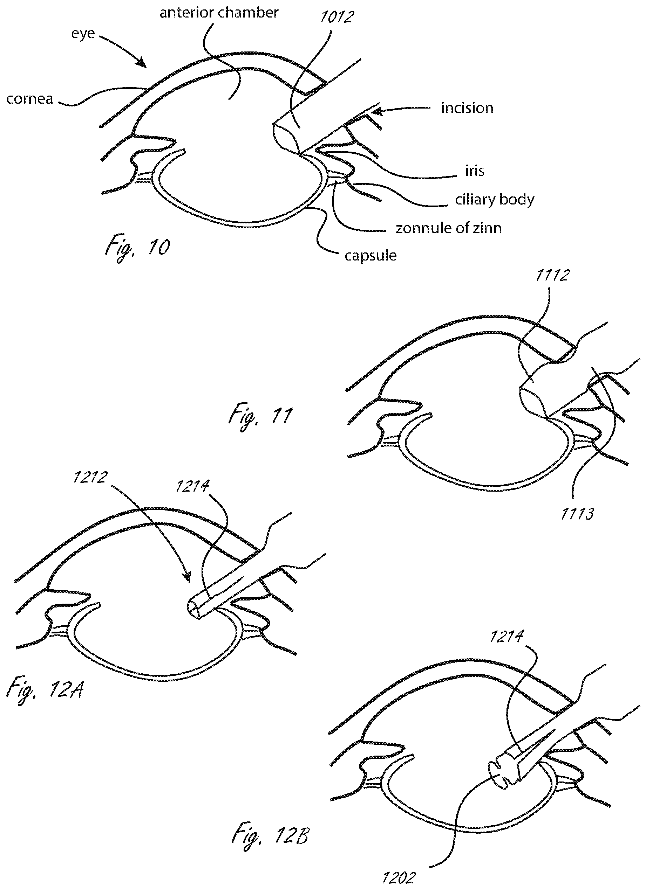

FIG. 10 illustrates a distal tip portion 1012 of a funneling insert (not shown) configured in accordance with an embodiment of the present technology. As can be seen in FIG. 10, the eye includes various structures such as an anterior chamber, an iris, a ciliary body, a zonnule of zinn, a capsule, and a cornea. The incision can be made in the cornea in accordance with techniques known to those of skill in the art. The distal tip portion 1012 can be inserted through the incision to a location adjacent or near the capsule to deliver an AIOL (not shown) into the capsule. In the illustrated embodiment, the distal tip portion 1012 has a cylindrical shape, e.g., similar to the distal tip portion 712 of FIG. 7. In other embodiments, however, the distal tip portion 1012 can have a different shape.

FIG. 11 illustrates a distal tip portion 1112 of a funneling insert (not shown) configured in accordance with another embodiment of the present technology. Similar to the distal tip portion 812 of FIG. 8, a constriction 1113 can be formed in the proximal portion of the distal tip portion 1112. As shown in FIG. 11, when the distal tip portion 1112 is inserted into the eye, the constriction 1113 can be positioned adjacent to and/or interface with the incision. The narrowed geometry of the constriction 1113 can reduce the amount of force applied by the distal tip portion 112 to the incision site to reduce inadvertent tissue damage during AIOL delivery.

FIGS. 12A and 12B illustrate a distal tip portion 1212 of a funneling insert (not shown) configured in accordance with a further embodiment of the present technology. The distal tip portion 1212 includes a portion having a uniform reduced cross-sectional dimension that is configured for insertion through an incision in the eye. The distal tip portion 1212 also includes one or more slits 1214 formed made in the distalmost section. The slits 1214 can allow the portion of the distal tip portion 1212 distal to the incision to open up and/or expand within the eye during delivery of a lens 1202 (e.g., an AIOL). This approach can further reduce the incision size and/or injury to the eye during delivery.

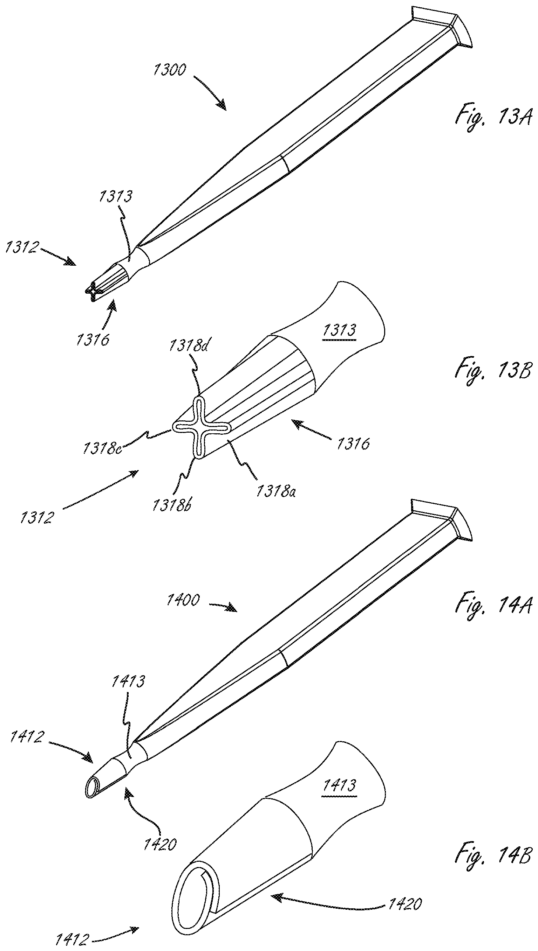

FIGS. 13A and 13B illustrate a funneling insert 1300 configured in accordance with another embodiment of the present technology. In the illustrated embodiment, the distal tip 1312 of the funneling insert 1300 comprises a constriction 1313 (e.g., similar to funneling insert 800 of FIG. 8). The distal tip portion 1312 can include a tapered and/or folded portion 1316 distal to the constriction 1313. The folded portion 1316 can be connected to and/or formed as a unitary part with the constriction 1313. As illustrated in FIG. 13B, the folded portion 1316 can include a plurality of folds 1318a-d and/or crimps. In the illustrated example, the funneling insert 1300 includes four folds 1318a-d (collectively, 1318). In some embodiments, more folds may be used and, in some embodiments, fewer folds may be used. The folds 1318 can extend parallel to a longitudinal axis of the funneling insert 1300. In some embodiments, the folds 1318 extend in a helical pattern and/or a diagonal pattern with respect to the longitudinal axis of the funneling insert 1300. The folds 1318 can be formed, for example, by creasing, crimping, and/or otherwise deforming a tube of material. The tube may have a cylindrical, oval-shaped, frusto-conical, and/or polygonal cross-section prior to folding. In some embodiments, the folds 1318 can be configured to unfold to open the distal tip portion 1312 during AIOL delivery while avoiding or reducing the risk of injury to the entry wound through which the distal end of the funnel passes.

FIGS. 14A and 14B illustrate a funneling insert 1400 configured in accordance with another embodiment of the present technology. In the illustrated embodiment, the distal tip portion 1412 of funneling insert 1400 comprises a constriction 1413. The distal tip portion 1412 can include a tapered and/or rolled portion 1420 (FIG. 14B) distal to the constriction 1413. The rolled portion 1420 can be connected to and/or formed as a unitary part with the constriction 1413. As illustrated in FIG. 14B, the rolled portion 1420 can have a tapered and/or beveled shape. The rolled portion 1420 can be formed, for example, by cutting and overlapping a portion of a tube. The tube may have a cylindrical, oval-shaped, and/or other cross-section prior to cutting and/or overlapping. In some embodiments, the rolled portion 1420 can be configured to unfurl to open the distal tip portion 1412 for AIOL delivery while avoiding or reducing the risk of injury to the entry wound through which the distal end of the funnel passes.

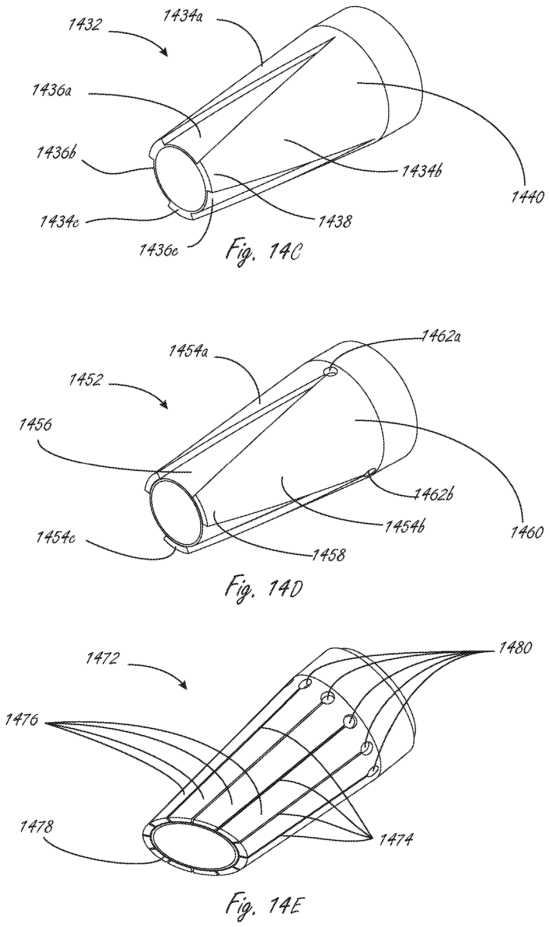

FIG. 14C is a closeup view of a distal tip portion 1432 of a funneling insert (not shown) configured in accordance with an embodiment of the present technology. The distal tip portion 1432 comprises a plurality of segments or flaps 1434a-c connected to each other by a plurality of membrane portions 1436a-c. In the illustrated example, the distal tip portion 1432 includes three flaps 1434a-c (collectively, 1434) and three membrane portions 1436a-c (collectively, 1436). In some embodiments, more flaps and/or membrane portions may be used, and in some embodiments, fewer flaps and/or membrane portions may be used. Each flap 1434 can have a tapered or trapezoidal shape, with a distal region 1438 of the flap 1434 being narrower than a proximal region 1440 of the flap 1434. Each membrane portion 1436 can have a triangular shape and can be positioned between a corresponding pair of flaps 1434. In some embodiments, one or more of the membrane portions 1436 have a trapezoidal shape or other shape. In some embodiments, the flaps 1434 and membrane portions 1436 are integrally formed with each other from a single material (e.g., by cutting, engraving, etching, molding, etc.). The membrane portions 1436 can be thinner and/or more compliant than the flaps 1434. The membrane portions 1436 can stretch and/or deform (e.g., plastically or elastically deform) to allow the flaps 1434 to move apart from each other and/or move away from the central longitudinal axis of the distal tip 1432 to open the distal tip portion 1432. For example, the membrane portions 1436 and flaps 1434 can deform, deflect, stretch, and/or otherwise move in response to passage of an AIOL through the distal tip portion 1432.

FIG. 14D is a closeup view of a distal tip portion 1452 of a funneling insert (not shown) configured in accordance with another embodiment of the present technology. The distal tip portion 1452 comprises a plurality of segments or flaps 1454a-c connected to each other by a membrane 1456. The flaps 1454 can be substantially similar to or the same as the flaps 1434 described above. In the illustrated example, the distal tip portion 1452 includes three flaps 1454a-c (collectively, 1454). In some embodiments, more flaps may be used, and in some embodiments, fewer flaps may be used. Each flap 1454 can have a tapered or trapezoidal shape, with the distal region 1458 of the flap 1454 being narrower than the proximal region 1460 of the flap 1454. The membrane 1456 can have a tubular shape and can be coupled to the inner surfaces of the flaps 1454 (e.g., by adhesives, bonding, fasteners). In some embodiments, the membrane 1456 has a tapered (e.g., frustoconical) shape wherein a distal end of the membrane 1456 has a smaller diameter or width than a proximal end of the membrane 1456. In some embodiments, the membrane 1456 is made from a compliant material that can stretch and/or deform (e.g., plastically or elastically deform) to allow the flaps 1454 to move away from each other and/or away from the central longitudinal axis of the distal tip portion 1452, thereby opening the distal tip portion 1452. The membrane 1456 can stretch and/or deform independently of the flaps 1454, thus reducing the likelihood of damage during delivery (e.g., from stress concentration).

Optionally, the distal tip portion 1452 can include a plurality of strain relief cutouts, indentations, or apertures 1462 (e.g., apertures 1462a-b). The strain relief apertures 1462 can be located on both sides of the proximal region 1460 of each flap 1454. The strain relief apertures 1462 can be shaped and/or sized to facilitate movement of the flaps 1454 apart from each other and/or away from the central longitudinal axis of the distal tip. For example, the strain relief apertures 1462 can have a circular or elliptical shape. In some embodiments, the strain relief apertures 1462 can inhibit or prevent cracking, splitting, and/or other damage when the distal tip portion 1452 is opened (e.g., due to stress concentration).

FIG. 14E is a closeup view of a distal tip portion 1472 of a funneling insert (not shown) configured in accordance with a further embodiment of the present technology. In the illustrated embodiment, the distal tip portion 1472 comprises a plurality of slits 1474. The slit 1474 can be distributed circumferentially around the distal tip portion 1472. The slits 1474 can extend substantially parallel to each other and along the longitudinal axis of the distal tip portion 1472 so as to form a plurality of elongate flaps 1476. Although the illustrated example includes twelve slits 1474 and twelve flaps 1476, the number of slits 1474 and flaps 1476 can be varied as desired (e.g., the distal tip portion 1472 can include more slits 1474 and flaps 1476, or fewer slits 1474 and flaps 1476). In some embodiments, the distal tip portion 1472 includes an internal membrane 1478 having a tubular shape and coupled to the inner surfaces of the flaps 1476. In some embodiments, the membrane 1478 has a tapered and/or frustoconical shape. The membrane 1478 can be made from a compliant material that stretches and/or deforms (e.g., plastically or elastically deforms) to allow the flaps 1476 to move away from each other and/or away from the central longitudinal axis of the distal tip portion 1472 to open the distal tip portion 1472. Optionally, the distal tip portion 1472 can include a plurality of strain relief cutouts, indentations, or apertures 1480 each located at a proximal end of a corresponding slit 1474. The strain relief apertures 1480 can facilitate movement of the flaps 1476 (e.g., similar to the strain relief apertures 1462 of FIG. 14D).

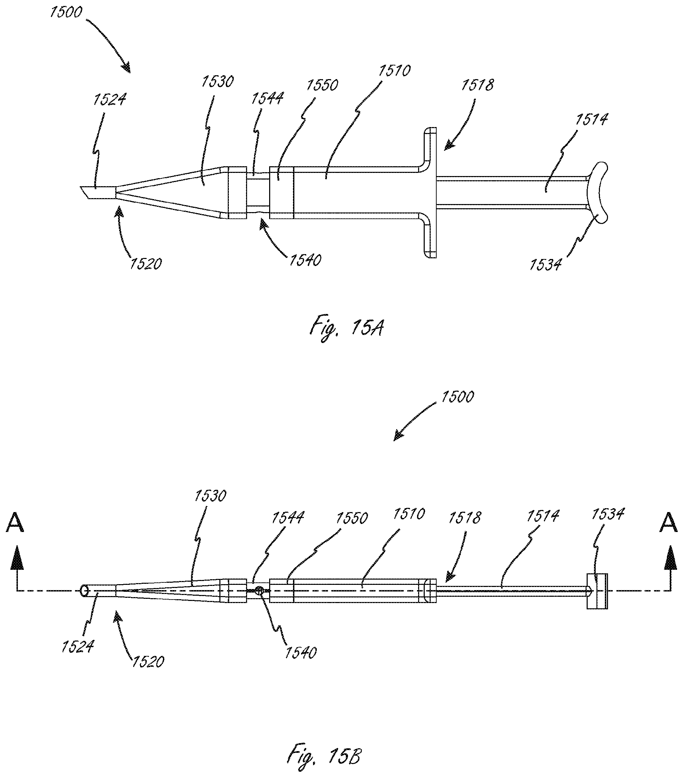

FIGS. 15A-15E illustrate an AIOL delivery system 1500 configured in accordance with an embodiment of the present technology. Referring first to FIG. 15A, the delivery system 1500 can include an injector body 1510 and a plunger 1514 operably connected to the injector body. The injector body 1510 can include any of the features of any of the injector bodies/funneling inserts described herein (e.g., with respect to FIGS. 1-3 and 7-14E). For example, as illustrated in FIG. 15A, the injector body 1510 can include a proximal end portion 1518 and a distal end portion 1520. The distal end portion 1520 can include a distal tip portion 1524. The distal tip portion 1524 can be similar to or the same as any of the distal tip portions described herein (e.g., with respect to FIGS. 1-3 and 7-14E. In some embodiments, the distal end portion 1520 of the injector body 1510 includes a tapered portion 1530. The tapered portion 1530 can have a tapered shape which transitions from a wider proximal end to a narrower distal end. In some embodiments, the distal end of the tapered portion 1530 has approximately the same cross-section as the distal tip portion 1524.

The plunger 1514 can be sized and shaped to extend through the proximal end portion 1518 (e.g., through an opening thereof) of the injector body 1510. The plunger 1514 can include a proximal end 1534 having an engagement feature. The engagement feature can be sized and/or shaped to facilitate user input to the plunger 1514. For example, the engagement feature can be an indentation, dimple, saddle, and/or some other feature configured to facilitate engagement between the user (e.g., a user's thumb or finger) and the plunger 1514. In some embodiments, the plunger 1514, or some portion thereof, can be configured to move in response to mechanical and/or electromechanical input. In some embodiments, the plunger 1514 is threaded or otherwise configured to engage with the injector body 1510. The plunger 1514 can include any of the features of any of the plungers/pistons described herein (e.g., with respect to FIGS. 1-6).

As illustrated in FIG. 15B, the injector body 1510 or some other portion of the delivery system 1500 can include one or more ports 1540. For example, the injector body 1510 can include first and second ports 1540 positioned along the length of the injector body 1510. The ports 1540 can be configured to allow access to an interior of the injector body 1510 or some other portion of the delivery system 1500. In some embodiments, the ports 1540 are positioned in a necked or narrowed portion 1544 of the injector body 1510. In some embodiments, the injector body 1510 does not include a necked or narrowed portion and the ports 1540 extend through a portion of the injector body 1510 between the proximal end portion 1518 and the distal end portion 1520.

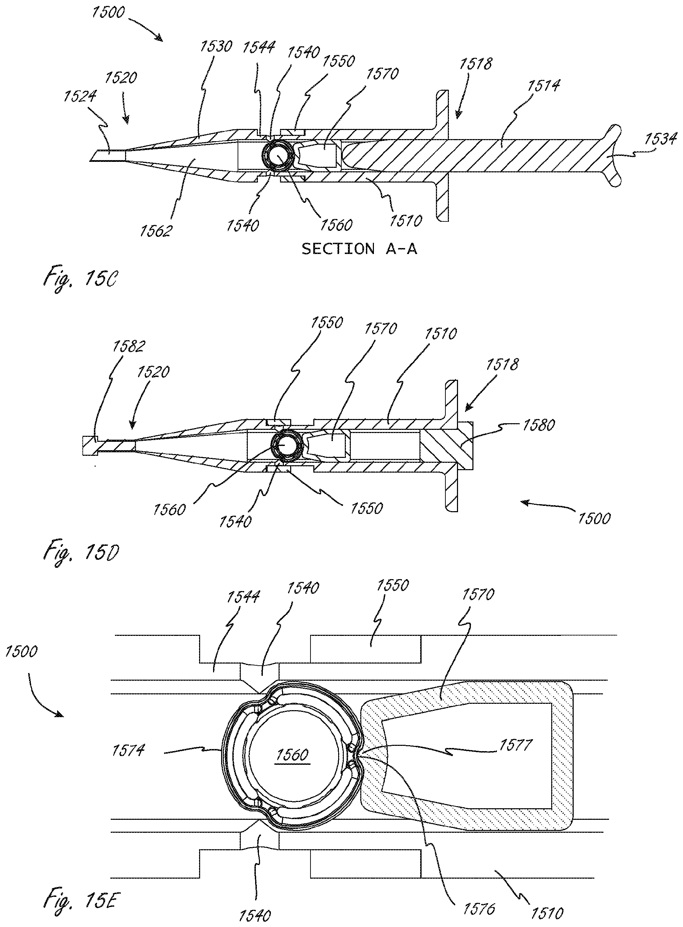

The delivery system 1500 can include one or more seals, valves, and/or some other structure(s) configured to selectively open, close, cover, and/or uncover the one or more ports 1540. In the illustrated embodiment, for example as shown in FIGS. 15C and 15D, a seal 1550 can be movably connected to the injector body 1510. In a first position, as illustrated in FIGS. 15A-15C, the seal 1550 can be positioned away from the ports 1540. In a second position, as illustrated in FIG. 15D, the seal 1550 can be positioned covering the one or more ports 1540. The seal 1550 can include one or more detent structures or other features configured to engage with the ports 1540 to reduce the risk of accidental unsealing (e.g., uncovering) of the ports 1540 while allowing for intentional movement of the seal 1550 away from the ports 1540.

The one or more ports 1540 can be configured, when opened, to allow for injection and/or insertion of material into the interior of the injector body 1510. Such material can include, for example, ophthalmic viscoelastic device (OVD) material or other appropriate materials. Such material can be introduced, for example, via a syringe or other fluid injection device. In some embodiments, the ports 1540 are positioned along the length of the injector body 1510 distal to a storage position of the AIOL 1560. Positioning the ports 1540 distal to the AIOL 1560 can allow for injection of material between the AIOL 1560 in the distal tip portion 1524. In some embodiments, the inner channel 1562 of the injector body 1510 is sized and shaped such that the AIOL 1560 inhibits or prevents passage of OVD material from a distal side of the AIOL 1560 to a proximal side of the AIOL 1560 within the injector body 1510. Introducing OVD material or other similar material to distal portion of the injector body 1510 before implantation of the AIOL 1560 can reduce the risk of damage to the eye as the AIOL 1560 is passed through the distal tip portion 1524 of the injector body 1510. Use of OVD material or other similar materials can help to maintain the anterior chamber of the eye, as well as protect the corneal endothelium during implantation of the AIOL.

Referring again to FIGS. 15C and 15D, the delivery system 1500 can include a flexible member 1570 (e.g., a cushion, pillow, and/or some other structure configured to at least partially deform in response to an exterior force). The flexible member 1570 can be positioned within the injector body 1510. In some embodiments, the flexible member 1570 is resilient. The flexible member 1570 can constructed as a solid and/or uniform structure. For example, the flexible member 1570 can be constructed from a hydrogel material. In some embodiments, the flexible member 1570 is hollow and/or filled with the material different from the material forming the outer wall the flexible member 1570. For example, the flexible member 1570 can be formed from a flexible outer shell filled with a filler material, such as a liquid, gel (e.g., a hydrogel), gas, and/or some combination thereof. In some embodiments, the filler material used to fill the flexible member 1570 is more compliant (e.g., more flexible/less viscous) than the material used to form the outer shell.

The flexible member 1570 can be configured to reduce the likelihood of damage to the AIOL 1560 before or during implantation. For example, the flexible member 1570 can reduce the likelihood of damage imparted on the AIOL 1560 from the plunger 1514. In some embodiments, use of the flexible member 1570 allows for improved surface area contact between structure pushing the AIOL 1560 and AIOL 1560. In other words, the flexible member 1570 can be configured to contact the AIOL 1560 over a large portion of the AIOL 1560 surface area as observed in the distal direction from the proximal opening of the injector body 1510. As the inner channel 1562 of the injector body 1510 narrows toward the distal tip portion 1524, the flexible member 1570 can also narrow. The flexibility and/or compressibility of the flexible member 1570 can therefore allow for contact between the flexible member 1570 in the AIOL 1560 that is substantially equal to the cross-sectional area of the inner channel 1562 as the AIOL 1560 passes through the inner channel 1562 to the distal tip portion 1524.

As illustrated in FIG. 15E, the AIOL 1560 can include one or more circumferential portions 1574 (e.g., flexible portions) defined by indentations 1576 along the perimeter of the AIOL 1560. In some embodiments, the indentations 1576 correspond to relatively stiff portions of the AIOL 1560. The flexible member 1570 can include a protrusion or other engagement feature 1577 configured to engage with one of the indentations 1576 of the AIOL 1560. In the illustrated embodiment, wherein the AIOL 1560 has an odd number (e.g., three, five, or some other number) of indentations 1576, aligning an indentation 1576 of the AIOL 1560 with engagement features 1577 of the flexible member 1570 can increase the likelihood that a flexible portion 1574 of the AIOL 1560 is positioned in the distal direction. Positioning a flexible portion 1574 of the AIOL 1560 on a distal side of the AIOL 1560 can improve compression of the AIOL 1560 and reduce stress on the AIOL 1560 as the AIOL 1560 passes through the inner channel 1562 the injector body 1510, and through an opening of the distal tip portion 1524.

Returning to FIG. 15D, the delivery system 1500 is illustrated in a storage or shipping configuration. In the storage configuration, the seal 1550 can be positioned in the second position sealing the ports 1540. In some embodiments, the plunger 1514 is removed. For example, a plug 1580 or other ceiling structure positioned within the opening in the proximal end portion 1518 of the injector body 1510. In some embodiments, a distal plug 1582 is positioned to seal the distal tip portion 1524. Instead of or in addition to the plugs 1580, 1582, in some embodiments foil, polymeric, and/or other thin/removable material may be placed over the opening of the distal tip portion 1524 and/or over the opening in the proximal end portion 1518 of the injector body 1510. Preferably, the inner channel 1562 of the injector body 1510 is at least partially filled with a buffer material. The buffer material can be, for example, saline solution or other material configured to maintain the AIOL 1560 and other portions (e.g., the flexible member 1570) of the delivery system 1500 in a desired condition (e.g., hydrated).

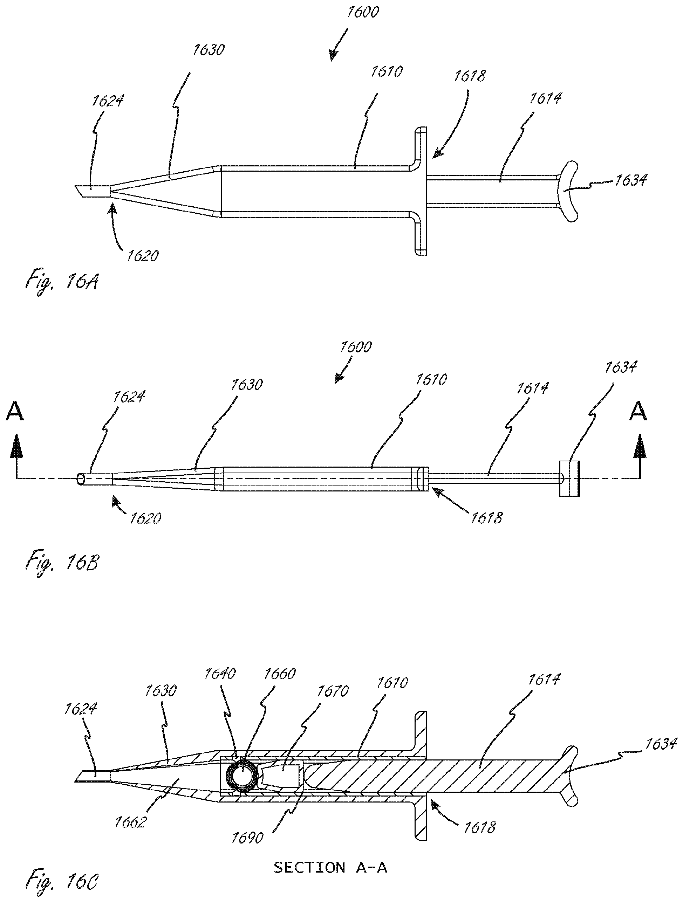

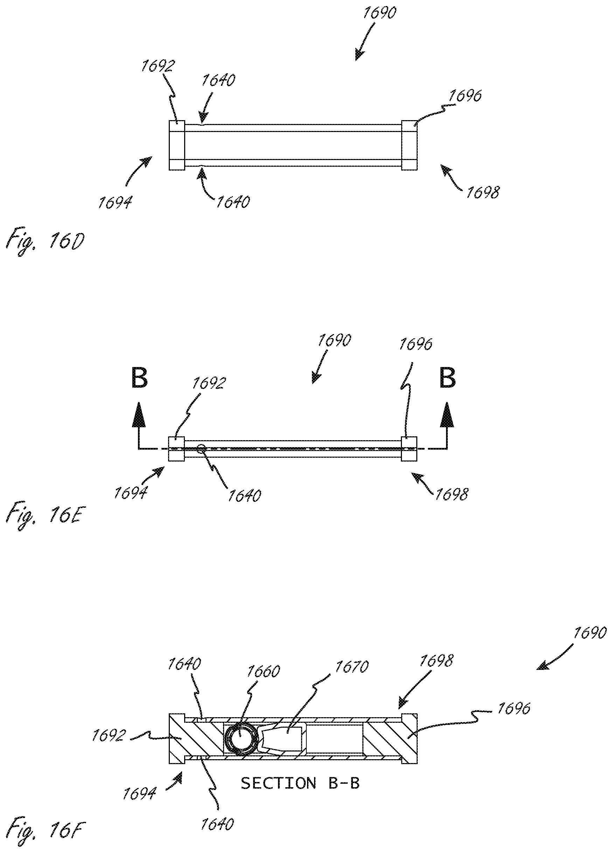

FIGS. 16A-16F illustrate an AIOL delivery system 1600 configured in accordance with another embodiment of the present technology. The delivery system 1600 can be generally similar to the delivery system 1500 described above with respect to FIGS. 15A-15E. Accordingly, like numbers (e.g., injector body 1510 versus injector body 1610) are used to identify similar or identical structures and discussion of the delivery system 1600 illustrated in FIGS. 16A-16F will be limited to those features that differ from the embodiment discussed with respect to FIGS. 15A-15E.

Referring to FIGS. 16A and 16B, the injector body 1610 may lack ports. In some embodiments, the injector body 1610 of the delivery system 1600 does not include any necked or narrowed portions. As illustrated in FIG. 16C, the delivery system 1600 can include a cartridge 1690 positioned within the injector body 1610. The cartridge 1690 may be removed from the injector body 1610 after use. In some embodiments, the cartridge 1690 can be shipped and stored separately from the injector body 1610 and/or the plunger 1614 and inserted into the injector body 1610 prior to use. One or both of the AIOL 1660 and the flexible member 1670 may be positioned within the cartridge 1690. In some embodiments, the AIOL 1660 and/or the flexible member 1670 are the same as or similar to the AIOL 1560 and flexible member 1570, respectively, described above.

As illustrated in FIGS. 16D and 16E, the cartridge 1690 can include one or more ports 1640. The one or more ports 1640 can operate in a manner similar to or the same as the ports 1540 described above. In some embodiments, delivery system 1600 can include one or more plugs configured to engage with the cartridge when the cartridges in a storage and/or shipping configuration. For example, the delivery system 1600 can include a first plug 1692 configured to seal the distal end 1694 of the cartridge 1690 and to seal the one or more ports 1640. A second plug 1696 can be positioned in or on the proximal end 1698 of the cartridge 1690. In some embodiments, in addition to or instead of the plugs, caps, seals (e.g., foil, polymer, and/or other seals), and/or other structures may be used to seal an interior of the cartridge 1690. Preferably, a buffering solution of (e.g. saline solution or other material) at least partially fills an interior of the cartridge 1690 when the cartridge 1690 is stored and/or shipped.

During use, the cartridge 1690 may be inserted into the injector body 1610. Preferably, the plugs, caps, seals, and other ceiling structures are removed prior to insertion of the cartridge 1690 into the injector body 1610. The plunger 1614 may then be introduced into the proximal end of the injector body 1610 and used in a manner similar to the same as the plunger 1514 described above with respect to FIGS. 15A-15E.

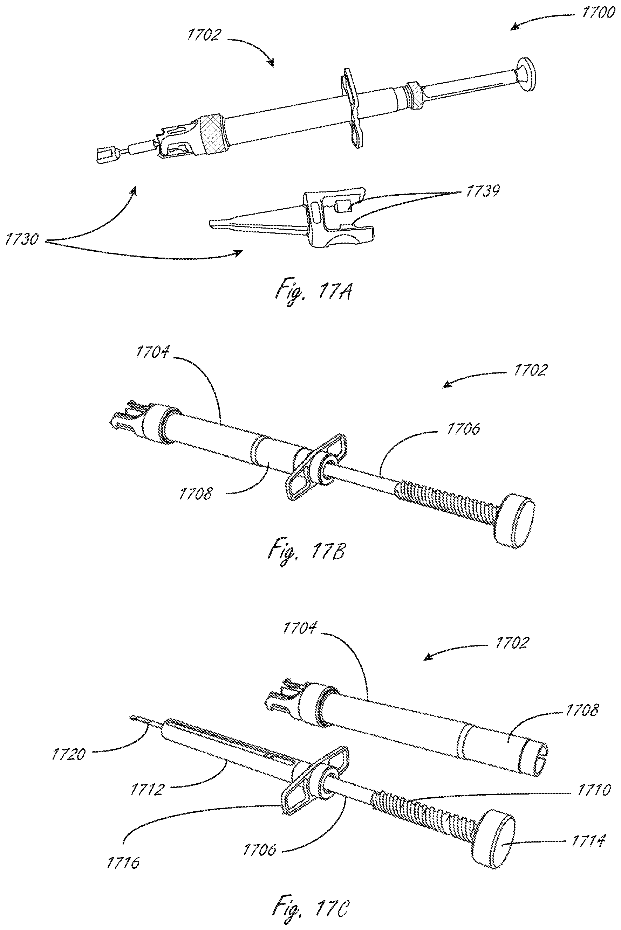

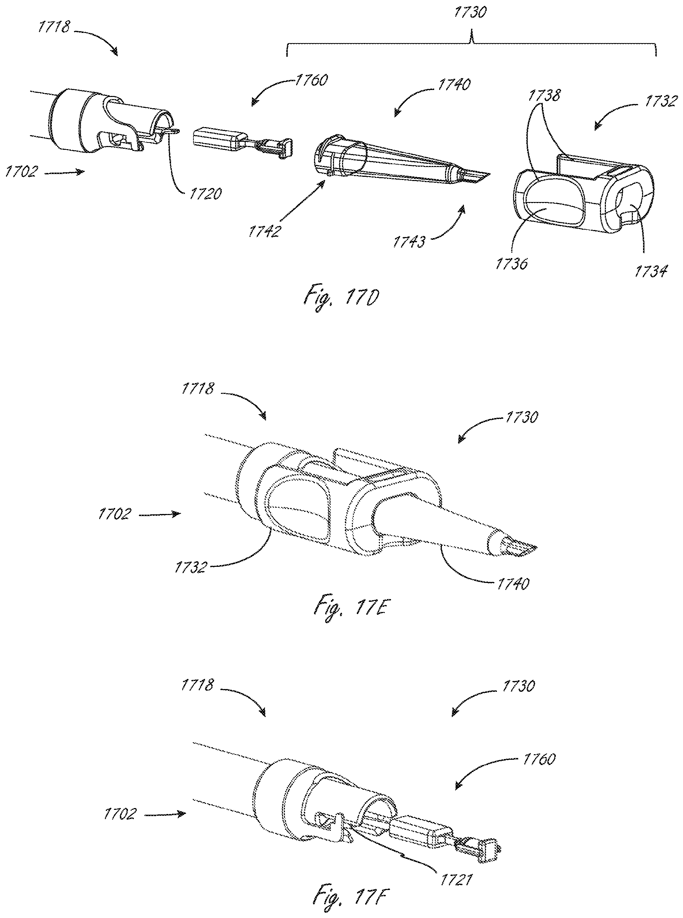

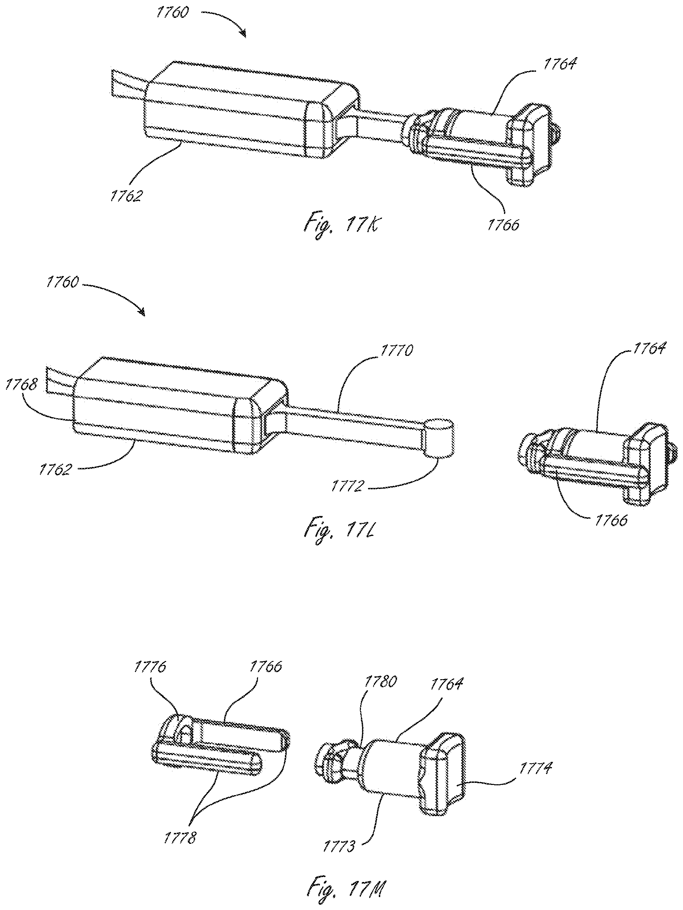

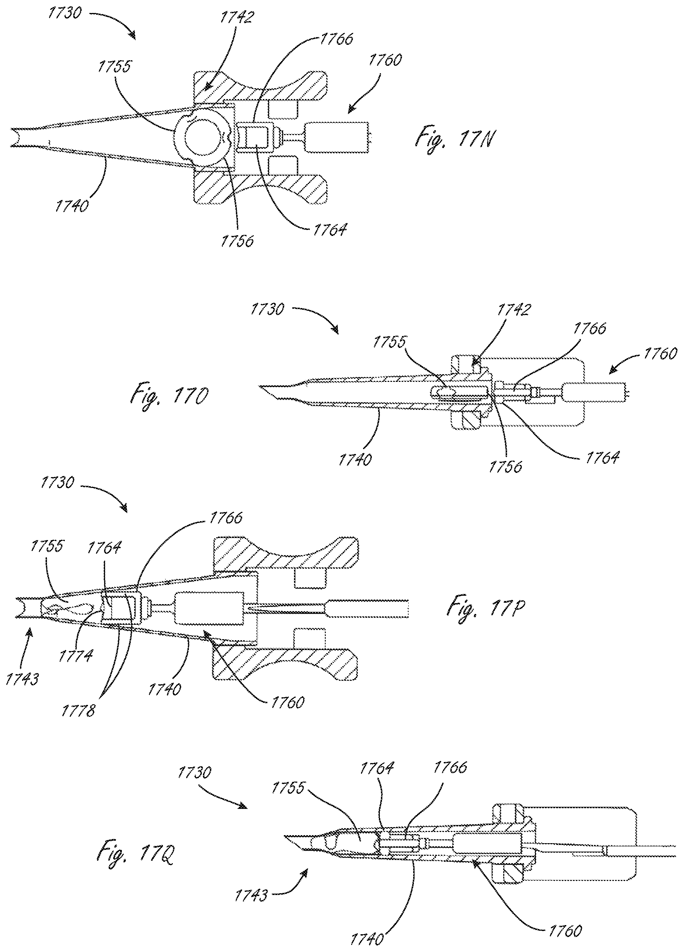

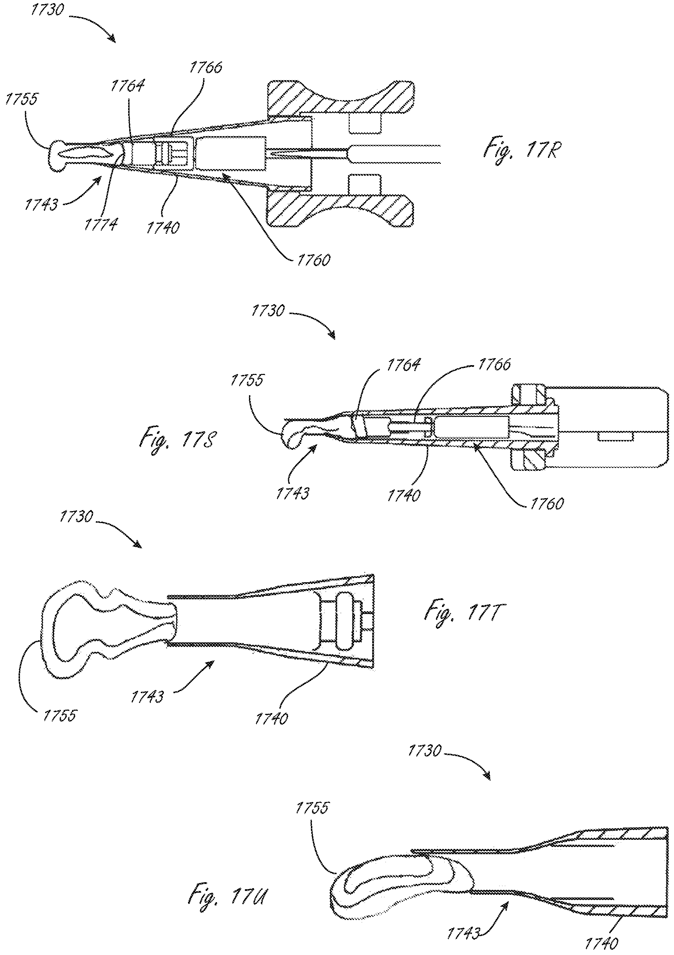

FIGS. 17A-17U illustrate an embodiment of an AIOL delivery system 1700 configured in accordance with a further embodiment of the present technology. The delivery system 1700 includes an injector 1702 (FIGS. 17B and 17C) and a tip assembly 1730 (FIGS. 17D-17M), as described in further detail below. The delivery system 1700 can be used to deliver an AIOL into a patient's eye as described in further detail below (FIGS. 17N-17U). In some embodiments, the injector 1702 may include one or more features similar to an IOL injector. In other embodiments the injector 1702 can be any device suitable for delivering a lens or other implantable optical component into the eye. The tip assembly 1730 can be operably coupled to the injector 1702 to adapt the injector 1702 for AIOL delivery in accordance with the embodiments described herein. As described in detail below, the various components of the tip assembly 1730 can be configured to controllably and reliably deliver an AIOL into the eye via a relatively small incision, while reducing or avoiding stresses on the AIOL likely to lead to rupture or other damage.

FIGS. 17B and 17C illustrate the injector 1702 of the delivery system 1700 when assembled (FIG. 17B) and when disassembled (FIG. 17C). The injector 1702 can be operated by a user to actuate delivery of an AIOL into a patient's eye. In the illustrated embodiment, the injector 1702 includes a body portion 1704 and a plunger 1706. The plunger 1706 can be configured to move distally relative to the body portion 1704. In some embodiments, for example, the body portion 1704 includes an elongate tube 1708 sized and/or shaped to receive the plunger 1706 therein. The plunger 1706 can include an elongate shaft 1710 slidably positioned within a barrel 1712. When the injector 1702 is assembled (FIG. 17B), the barrel 1712 can be positioned within the tube 1708, with a portion of the shaft 1710 extending proximally outwards from the body portion 1704. The plunger 1706 can be actuated by a user to move the shaft 1710 distally relative to the barrel 1712 and body portion 1704. The plunger 1706 can further include a distal extension 1720 configured to engage and transmit the actuation force to another component of the delivery system 1700, as described in greater detail below.

Optionally, the injector 1702 can include features that allow the injector 1702 to be held and/or operated with one hand. In some embodiments, for example, the plunger 1706 includes a first engagement feature 1714 and a second engagement feature 1716 sized and/or shaped to facilitate actuation of the plunger 1706. For example, the first engagement feature 1714 (e.g., a flange, thumb rest, or other structure) can be located on the shaft 1710 for engagement with a user's thumb and the second engagement feature 1716 (e.g., a flange, loop, or other structure) can be located on the barrel 1712 for engagement with the user's fingers. In other embodiments, however, the first and second engagement features 1714 and 1716 may have different configurations, and/or may not include one or both of these engagement features.

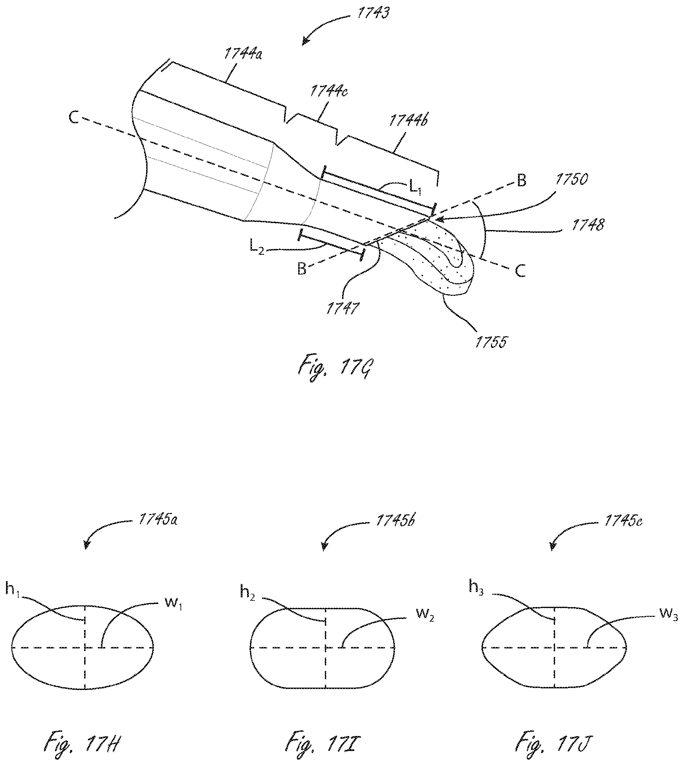

FIGS. 17D-17K illustrate various components of the tip assembly 1730 of the delivery system 1700. For example, the tip assembly 1730 can be coupled to the distal portion 1718 of the injector 1702 (FIGS. 17D-17F). The tip assembly 1730 can include an adapter 1732 (FIGS. 17D and 17E), an injector tip 1740 (FIGS. 17D, 17E, and 17G-17I), and a plunger assembly 1760 (FIGS. 17D, 17F, and 17K-17M). In the illustrated embodiment, the adapter 1732, injector tip 1740, and plunger assembly 1760 are discrete components that are attached to each other (e.g., via interference fit, mating features, adhesives, bonding, etc.) to assemble the tip assembly 1730. In other embodiments, however, some of these components can be integrally formed with each other as a single unitary structure (e.g., the adapter 1732 and injector tip 1740).

FIGS. 17D-17F illustrate the tip assembly 1730 and the distal portion 1718 of the injector 1702 in both an exploded view (FIG. 17D) and when assembled (FIGS. 17E-17F). Referring to FIGS. 17D-17F together, the tip assembly 1730 includes an adapter 1732 configured to couple to the injector 1702, an injector tip 1740 configured to receive an AIOL (not shown), and a plunger assembly 1760 configured to push the AIOL out of the injector tip 1740 when the injector 1702 is actuated. The tip assembly 1730 can be sized and shaped such that it can be coupled to or otherwise engaged with the distal portion 1718 of the injector 1702. For example, FIGS. 17E and 17F illustrate the tip assembly 1730 when coupled to the distal portion 1718 of the injector 1702 (the adapter 1732 and injector tip 1740 are omitted in FIG. 17F for clarity).

Referring to FIGS. 17D and 17E together, the adapter 1732 can be sized and/or shaped to couple the injector tip 1740 and/or plunger assembly 1760 to the distal portion 1718 of the injector 1702. For example, the adapter 1732 can include a distal aperture 1734 configured to receive and/or mate with a corresponding portion the injector tip 1740 (e.g., a proximal portion 1742). The adapter 1732 can further include an engagement portion 1736 that couples to the distal portion 1718 of the injector 1702 to secure the injector tip 1740 to the injector 1702. For example, the engagement portion 1736 can include a pair of proximally-extending side walls 1738 shaped to engage the distal portion 1718 of the injector 1702. The side walls 1738 can optionally include features configured to couple to mating features on the distal portion 1718 of the injector 1702. For example, the side walls 1738 can include one or more tabs 1739 (FIG. 17A) shaped to be received within one or more slots 1721 in the distal portion 1718 of the injector 1702 (FIG. 17F). During assembly, the adapter 1732 can be slid over the injector 1702 so that the tabs 1739 are positioned within the slots 1721. The adapter 1732 can then be rotated relative to the injector 1702 (or vice-versa) to lock the tabs 1739 within the slots 1721. In other embodiments the adapter 1732 and injector 1702 can include different types of mating features, such as protrusions, pins, groove, holes, threading, etc.

Referring to FIGS. 17D, 17E, and 17G-17I together, the injector tip 1740 can be a hollow structure configured to receive an AIOL (e.g., AIOL 1755 shown in FIG. 17G) and/or any other components useful for AIOL delivery (e.g., OVD material). As shown in FIG. 17D, the injector tip 1740 includes a proximal portion 1742 and a distal portion 1743. The proximal portion 1742 can be configured to couple to an injector 1702 (e.g., directly or indirectly via adapter 1732) and the distal portion 1743 can be configured to be inserted at least partially into an incision in the eye. The injector tip 1740 can include any of the features of any of the injector bodies/funneling inserts described herein (e.g., with respect to FIGS. 1-3 and 7-16F) and/or the distal portion 1743 can include any of the features of any of the distal portions or distal tip portions described herein (e.g., with respect to FIGS. 1-3 and 7-16F). For example, in some embodiments, the injector tip 1740 has a tapered shape such that the proximal portion 1742 of the injector tip 1740 is wider than the distal portion 1743 of the injector tip 1740. For example, the distal portion 1743 can have a width less than or equal to about 3.5 mm, 3.25 mm, 3 mm, 2.75 mm, 2.5 mm, 2.25 mm, or 2 mm. The proximal portion 1742 can have a width greater than or equal to about 5 mm, 5.5 mm, 6 mm, 6.5 mm, 7 mm, 7.5 mm, 8 mm, 8.5 mm, 9 mm, 9.5 mm, 10 mm, 10.5 mm, 11 mm, 11.5 mm, or 12 mm.

The geometry of the injector tip 1740 or at least a portion thereof (e.g., distal portion 1743 (FIG. 17G)) can be configured to improve delivery of the AIOL into the eye. For example, in some embodiments, the geometry of the distal portion 1743 is configured to compress an AIOL from an uncompressed resting configuration into a compressed delivery configuration. In the delivery configuration, the AIOL can have a folded and/or furled shape suitable for delivery into the eye via a small incision. For example, the compressed configuration can be suitable for delivery via an incision having a length of about 2.0 mm, 2.5 mm, 3.0 mm, 3.5 mm, 4.0 mm, 4.5 mm, or 5.0 mm. The geometry of the distal portion 1743 can be configured to controllably and consistently compress an AIOL into the delivery configuration without rupturing or otherwise damaging the AIOL.

Referring to FIG. 17G, for example, the distal portion 1743 can include a first section 1744a, a second section 1744b distal to the first section 1744a, and a tapered section 1744c connecting the first section 1744a and second section 1744b. The first section 1744a and second section 1744b can each have an elongated shape with a uniform or generally uniform cross-sectional geometry (e.g., with respect to area, diameter, circumference, width, shape, etc.). The first section 1744a can have a first cross-sectional dimension (e.g., area, diameter, circumference, width, height). The first cross-sectional dimension can be greater than or equal to a corresponding cross-sectional dimension of the AIOL 1755 when in a resting (e.g., uncompressed and/or undeformed) configuration. In some embodiments, the first cross-sectional dimension is less than or equal to a maximum dimension (e.g., a diameter) of the AIOL 1755. The second section 1744b can have a second cross-sectional dimension smaller than the first cross-sectional dimension. The second cross-sectional dimension can be less than the corresponding cross-sectional dimension of the AIOL 1755 in the resting configuration. The tapered section 1744c can have a decreasing cross-sectional dimension that provides a smooth, gradual transition between the first and second sections 1744a-b. Accordingly, as the AIOL moves from the first section 1744a into the second section 1744b via tapered section 1744c, the decreasing cross-sectional dimension can cause the AIOL to be gradually compressed (e.g., folded and/or furled) from the resting configuration into the delivery configuration.

Referring to FIGS. 17G through 17I together, the cross-sectional shape of at least a part of the distal portion 1743 (e.g., the second section 1744b and/or tapered section 1744c) can be configured to facilitate compression of the AIOL into the delivery configuration in a controlled and consistent manner. In some embodiments, for example, the second section 1744b is shaped to interface with the AIOL 1755 to cause the AIOL 1755 to fold, furl, or otherwise transition into the delivery configuration. For example, the second section 1744b can have a cross-sectional shape that is an elliptical shape 1745a (FIG. 17H), a modified elliptical shape 1745b (FIG. 17I), or a rounded hexagonal shape 1745c (FIG. 17J). The modified elliptical shape 1745b can be, for example, an ellipse having one or more flattened sides (e.g., flattened top and bottom sides). The rounded hexagonal shape 1745c can be, for example, a six-sided shape having rounded corners and flattened top and bottom sides. In some embodiments, the lateral sides of the elliptical shape 1745a, modified elliptical shape 1745b, and/or rounded hexagonal shape 1745c interface with the corresponding lateral portions of the AIOL 1755 to encourage them to fold and/or furl upwards or downwards into the delivery configuration.

The cross-sectional dimensions of the second section 1744b (e.g., width, height, diameter) can be configured to compress the AIOL for delivery via a relatively small incision (e.g., an incision less than 3.5 mm long). In some embodiments, for example, the maximum cross-sectional width of the second section 1744b (e.g., width w₁ of FIG. 17H, width w₂ of FIG. 17I, width w₃ of FIG. 17J) is smaller than the diameter of the AIOL in its resting configuration, such that the AIOL is constrained into the compressed delivery configuration when positioned within the second section 1744b. For example, the maximum cross-sectional width of the second section 1744b can be less than or equal to about 3.5 mm, 3.25 mm, 3 mm, 2.75 mm, 2.5 mm, 2.25 mm, or 2 mm. In some embodiments, the height of the second section 1744b (e.g., height h₁ of FIG. 17H, height h₂ of FIG. 17I, height h₃ of FIG. 17J) is less than or equal to about 3. 2.5 mm, 2.25 mm, 2 mm, 1.75 mm, or 1.5 mm.

Referring again to FIG. 17G, the second section 1744b can terminate in an angled or beveled end portion 1747, such that the plane of the end portion 1747 (represented by line B-B) is at a bevel angle 1748 relative to the longitudinal axis of the distal portion 1743 (represented by line C-C). The bevel angle 1748 can be selected to allow the AIOL to be quickly and controllably delivered into the eye in a desired position and/or orientation, e.g., without flipping or inverting. In some embodiments, the bevel angle 1748 is about 20 degrees, 25 degrees, 30 degrees, 35 degrees, 40 degrees, 45 degrees, 50 degrees, 55 degrees, or 60 degrees.

The length of the second section 1744b (e.g., as measured from the proximal-most portion of the second section 1744b to the distalmost tip 1750) can be selected to facilitate AIOL delivery into the eye. In embodiments where the end portion 1747 is beveled, the second section 1744b can have a maximum length Li and a minimum length L₂. In some embodiments, the length (e.g., Li and/or L₂) is sufficiently long to allow the second section 1744b to be inserted into the eye, yet sufficiently short to reduce the likelihood of injury to the eye (e.g., due to excessive insertion depth). For example, the length can be about 1 mm, 1.5 mm, 2 mm, 2.5 mm, 3 mm, 3.5 mm, 4 mm, 4.25 mm, 4.3 mm, 4.35 mm, 4.4 mm, 4.45 mm, 4.5 mm, 4.55 mm, 4.6 mm, 4.65 mm, 4.7 mm, 4.75 mm, 4.8 mm, 4.85 mm, 4.9 mm, 4.95 mm, 5 mm, 5.25 mm, 5.5 mm, or 6 mm. In some embodiments, the minimum length L₂ is less than or equal to 1.5 mm, and the maximum length Li is greater than 1.5 mm.

In some embodiments, the length of the second section 1744b (e.g., Li and/or L₂) is configured to reduce compressive stresses on the AIOL while also allowing for sufficient insertion depth of the distal portion 1743 into the eye. For example, the length can be configured to reduce the portion of the AIOL that is compressed within the second section 1744b at any given point in time during delivery into the patient's eye. The geometry of the second section 1744b can be configured based on the size of the AIOL. For example, the length can be shorter than the diameter of the AIOL in order to reduce the portion of the AIOL that is compressed in the second section 1744b during delivery. Reducing the portion of the AIOL within second section 1744b at any given time during implantation can reduce stresses on the bonds of the AIOL exerted by fluid pressure within the AIOL in response to compression of the AIOL. In some embodiments, for example, the ratio of the length of the second section 1744b to the diameter of the AIOL is about 0.3, 0.35, 0.4, 0.45, 0.5, 0.55, 0.6, 0.65, 0.7, 0.75, or 0.8.

In some embodiments, the injector tip 1740 or a portion thereof (e.g., distal portion 1743) is configured to control a rotational angle of the AIOL as it moves distally through the injector tip 1740 and/or out the distal portion 1743 of the injector tip 1740. In some embodiments, the injector tip 1740 is configured to maintain the rotational angle of the AIOL during delivery such that the AIOL cannot rotate or such that the AIOL exhibits a relatively small amount of rotation (e.g., no more than 20 degrees, 15 degrees, 10 degrees, or 5 degrees of rotation). In some embodiments, the injector tip 1740 is configured to prevent or reduce AIOL rotation as the AIOL is ejected from the distal portion 1743 of the injector tip 1740. In other embodiments, however, the injector tip 1740 can be configured to rotate the AIOL during delivery. For example, the AIOL may be rotated to facilitate pushing through and out the distal portion 1743. In some embodiments, the injector tip 1740 is configured to cause the AIOL to rotate (e.g., 180 degrees) as it is ejected from the distal portion 1743 of the injector tip 1740.

The injector tip 1740 can be made of any suitable material, such as a polymer (e.g., polypropylene). In some embodiments, at least a portion of the injector tip 1740 includes a low-friction material or material coating (e.g., a hydrophilic material such as a hydrophilic polymer, resin, etc.). For example, the interior surface of the injector tip 1740 can include a low-friction material. In such embodiments, the low-friction material can reduce friction between the injector tip 1740 and the AIOL, such that the coefficient of friction is less than or equal to 10 N, 9 N, 8 N, 7 N, 6 N, 5 N, 4 N, 3 N, 2 n or 1 N. Alternatively or in combination, the external surface of the distal portion 1743 of the injector tip can include a low-friction material, e.g., to reduce friction between the distal portion 1743 and the tissues of the eye. The low-friction material can be applied to the injector tip 1740 using any suitable technique, such as coating, cross-linking, layering, depositing, or a combination thereof. The low-friction material can be, for example, a hydrophilic material (e.g., polyurethane, poly(vinylpyrrolidone), poly(ethylene oxide), poly(propylene oxide), polyacrylamide, methyl cellulose, polyacrylic acid, polyvinyl alcohol, polyvinyl ether, or combinations thereof). In some embodiments, the injector tip 1740 is made of a relatively hydrophobic material (e.g., polypropylene), and a hydrophilic low-friction material can be applied to the injector tip 1740 to increase the hydrophilicity thereof. Optionally, the injector tip 1740 can be pre-treated (e.g., plasma treated) to improve adhesion of the low-friction material to the injector tip 1740. The final thickness of the low-friction material can be varied as desired, e.g., about 5 microns to about 10 microns thick.

Referring to FIGS. 17D, 17F, and 17K-17M together, the plunger assembly 1760 is configured to push the AIOL distally out of the injector tip 1740 when actuated (e.g., by the injector 1702 shown in FIGS. 17D and 17F). In some embodiments, the plunger assembly 1760 is configured to maintain sufficient engagement with the AIOL even as the cross-sectional dimension of the injector tip 1740 decreases. This can be accomplished, for example, by altering the configuration of the plunger assembly 1760 as it moves distally through the injector tip 1740 so that at least a portion of the plunger assembly 1760 remains in contact with the AIOL. To accommodate the tapering of the injector tip 1740, the cross-sectional dimension (e.g., area, width) of the portion(s) of the plunger assembly 1760 in contact with the AIOL can decrease as the plunger assembly 1760 moves distally.

For example, as described in greater detail below, the plunger assembly 1760 can have a telescoping structure including an inner member (e.g., a plunger tip 1764) that is movable relative to an outer member (e.g., a frame structure 1766). The inner and outer members can have a combined cross-sectional dimension (e.g., width) that is narrower than the proximal portion 1742 of the injector tip 1740 but wider than the distal portion 1743 of the injector tip 1740. During a first stage of AIOL delivery, the plunger assembly 1760 can have a first configuration (e.g., a shortened and/or collapsed configuration) in which the inner member is positioned at least partially within the outer member so that these components engage and contact the AIOL together. During a second stage of AIOL delivery, the plunger assembly 1760 can move telescopically into a second configuration (e.g., an extended configuration) in which the inner member is positioned distally relative to the outer member and engages the AIOL without the outer member. As a result, the inner member contacts the AIOL during the most of or the entire process, while the outer member remains in contact until the injector tip 1740 becomes too narrow to allow further distal movement of the outer member.

Referring to FIGS. 17K (assembled view) and 17L-17M (exploded views), in some embodiments, for example, the plunger assembly 1760 includes a base 1762, a plunger tip 1764, and/or a frame structure 1766. The base 1762 can include an adapter portion 1768 and a stem 1770 (FIG. 17L) extending distally from the adapter portion 1768. The adapter portion 1768 can be coupled to the distal portion 1718 of the injector 1702 (e.g., to a distal extension 1720 of the plunger 1706 as illustrated in FIGS. 17C and 17F).

The plunger tip 1764 is configured to engage with and push against the AIOL. In some embodiments, the plunger tip 1764 is made of a flexible, compliant, and/or resilient material, such as silicone. The plunger tip 1764 can include an inner cavity (not shown) shaped to receive the distal end 1772 of the stem 1770, a body portion 1773, and an end portion 1774 shaped to engage the AIOL. The plunger tip 1764 can have a hammer-like shape, with the end portion 1774 being wider than the body portion 1773. The body portion 1773 can have an elongated (e.g., cylindrical) shape. The end portion 1744 can have a concave end surface shaped to receive and conform to the corresponding surface of the AIOL. In some embodiments, the end surface of the end portion 1774 has a generally polygonal (e.g., rectangular) or curved (e.g., circular or oval-shaped) face when viewed in the proximal direction from a point distal of the end portion 1774.

The frame structure 1766 can be configured to engage with and push against the AIOL in conjunction with the plunger tip 1764. The frame structure 1766 can be disposed at least partially around the plunger tip 1764. For example, the frame structure 1766 can include a ring 1776 and one or more prongs 1778 extending distally from the ring 1776. The prongs 1778 can be positioned on opposite sides of the ring 1776 and can extend parallel to each other along the longitudinal axis of the plunger assembly 1760. The ring 1776 can be seated around the plunger tip 1764 (e.g., around the body portion 1773 and/or a narrowed neck portion 1780). The prongs 1778 can extend along the body portion 1773 of the plunger tip 1764 and terminate at or near the end portion 1774. In some embodiments, the plunger tip 1764 and frame structure 1766 are arranged in a telescoping configuration, such that the plunger tip 1764 can be moved distally relative to the frame structure 1766.

FIGS. 17N-17U illustrate sequential steps in a delivery of an AIOL 1755 from the tip assembly 1730. Referring first to FIGS. 17N (top cross-sectional view) and 17O (side cross-sectional view), the AIOL 1755 is initially positioned within the injector tip 1740, e.g., within the proximal portion 1742. The proximal portion 1742 can be wider than the AIOL 1755 such that the AIOL 1755 is initially in an uncompressed resting configuration. The plunger assembly 1760 can be initially positioned proximal to the AIOL 1755 and injector tip 1740, with the plunger tip 1764 and frame structure 1766 near a proximal surface 1756 of the AIOL 1755.

Referring next to FIGS. 17P (top cross-sectional view) and 17Q (side cross-sectional view), when the plunger 1706 (not shown) is actuated, the actuation force can be transmitted to the plunger assembly 1760, causing the plunger assembly 1760 to advance distally into the injector tip 1740. The plunger tip 1764 and frame structure 1766 of the plunger assembly 1760 can both advance towards and engage the proximal surface 1756 of the AIOL 1755, thereby pushing the AIOL 1755 towards the distal portion 1743 of the injector tip 1740. For example, the plunger tip 1764 can contact the AIOL 1755 via the end portion 1774, while the frame structure 1766 can contact the AIOL 1755 via the prongs 1778. In some embodiments, the injector tip 1740 has a tapered shape with the distal portion 1743 being narrower than the AIOL 1755, such that the AIOL 1755 is compressed into a delivery configuration as it is pushed distally through the injector tip 1740. As previously described herein, the geometry of the injector tip 1740 or at least a portion thereof (e.g., distal portion 1743) can be selected to facilitate compression of the AIOL 1755 into the delivery configuration.

Referring to FIGS. 17R (top cross-sectional view) and 17S (side cross-sectional view), as actuation continues, the plunger assembly 1760 is advanced further through the injector tip 1740 towards the distal portion 1743. In some embodiments, the injector tip 1740 tapers to a width narrower than the combined width of the plunger tip 1764 and frame structure 1766, such that the frame structure 1766 is constrained by the inner walls of the injector tip 1740 and does not continue to move distally. The plunger tip 1764 can separate from the frame structure 1766 and continue to move towards the distal portion 1743, such that the AIOL 1755 is engaged and pushed distally by the plunger tip 1764 (e.g., via end portion 1774), and not by the frame structure 1766.

Referring to FIGS. 17T (closeup top cross-sectional view) and 17U (closeup side cross-sectional view), as delivery continues, the AIOL 1755 is pushed out of the distal portion 1743 of the injector tip 1740 and into the patient's eye. The AIOL 1755 can revert to its uncompressed resting configuration as it exits the distal portion 1743.

The system 1700 is expected to provide several advantages over conventional systems for AIOL delivery. For example, the tip assembly 1730 can be used to adapt many different types of injectors for AIOL delivery, thus allowing the methods herein to be performed with a wide variety of devices, including commercially available devices. As another example, the tapered geometry of the injector tip 1740 can gradually deform the AIOL from its normal state to a compressed state for delivery, without damaging the AIOL. The narrowed distal portion 1743 of the injector tip 1740 can also reduce the incision size in the patient's eye. Additionally, the telescoping configuration of the plunger tip 1764 and the frame structure 1766 can allow for smoother and more effective delivery of the AIOL through the tapered injector tip 1740 and into the eye, while maintaining sufficient surface area in contact with the AIOL during actuation to reduce the risk of damage thereto.

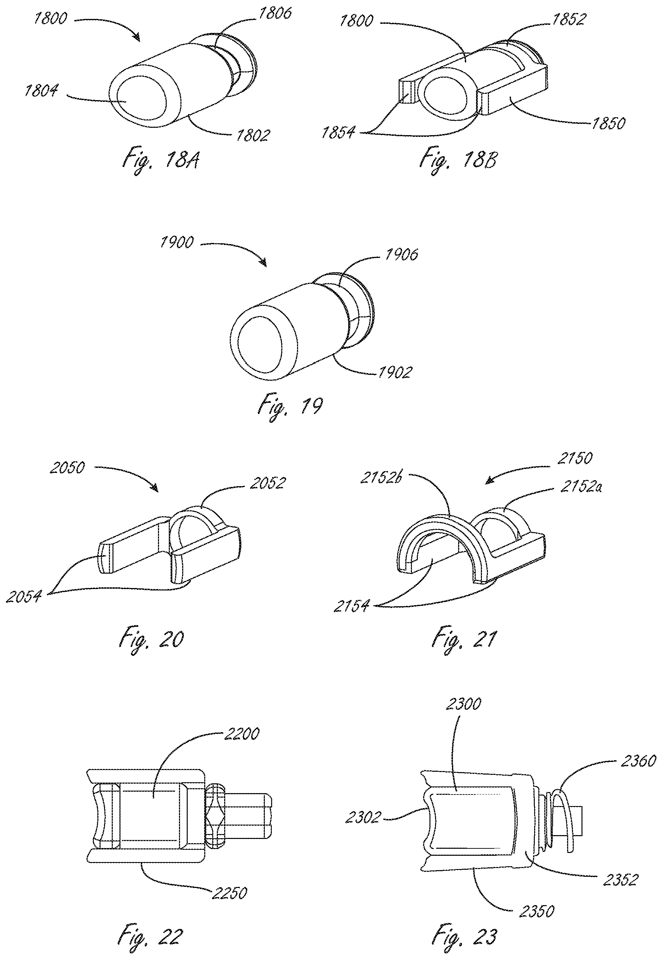

FIGS. 18A-29C illustrate various embodiments of plunger tips and frame structures capable of use with the AIOL delivery systems described herein (e.g., delivery system 1700 of FIGS. 17A-17U). The plunger tips and frame structures described herein can be similar to the plunger tip 1764 and frame structure 1766, respectively, described above with respect to FIGS. 17A-17U. Accordingly, the discussion of the embodiments illustrated in FIGS. 18-29C will be limited to those features that differ from the embodiments discussed with respect to FIGS. 17A-17U. It will be appreciated that the features of the embodiments described with respect to FIGS. 18A-29C can be combined with each other or with any other embodiment described herein.

FIGS. 18A and 18B illustrate a plunger tip 1800 and a frame structure 1850 of a plunger assembly. The plunger tip 1800 has an elongated body portion 1802 terminating in a distal end surface 1804. The body portion 1802 can have an elliptical cross-sectional shape. The end surface 1804 can be a curved (e.g., concave) surface configured to receive and conform to the corresponding surface of an AIOL. The frame structure 1850 can be positioned at least partially around the plunger tip 1800. The frame structure 1850 can include an elliptical ring 1852 and one or more prongs 1854 extending distally from the ring 1852. The ring 1852 can be seated around the plunger tip 1800 (e.g., around the body portion 1802 and/or a narrowed neck portion 1806). The prongs 1854 can extend along the body portion 1802 of the plunger tip 1800 and terminate at or near the end surface 1804.

FIG. 19 illustrates another embodiment of a plunger tip 1900. The plunger tip 1900 can be generally similar to the plunger tip 1800 described with respect to FIGS. 18A and 18B, except that the body portion 1902 has a circular cross-sectional shape. The plunger tip 1900 can include a narrowed neck portion 1906 for coupling to a frame structure (not shown).

FIG. 20 illustrates a frame structure 2050. The frame structure 2050 can be generally similar to the frame structure 1850 described with respect to FIG. 18B, except that the frame structure 2050 includes a circular ring 2052. A pair of prongs 2054 extend distally from the ring 2052. The frame structure 2050 can be coupled to a plunger tip having a circular cross-sectional shape, such as the plunger tip 1900 described with respect to FIG. 19.

FIG. 21 illustrates another embodiment of a frame structure 2150. The frame structure 2150 includes a first, proximal arcuate element 2152a and a second, distal arcuate element 2152b (collectively, 2152) connected to each other by a pair of prongs 2154. The arcuate elements 2152 can have a semi-circular or semi-elliptical shape. In some embodiments, the first arcuate element 2152a has a smaller diameter than the second arcuate element 2152b. In some embodiments, one or both of the arcuate elements 2152 extend over more than a 180° portion of a corresponding body portion (not shown). In some such embodiments, the frame structure 2150 is constructed from a flexible and/or resilient material configured to flex to allow mating (e.g., snap-fitting) of the frame structure 2150 with a body portion.

FIG. 22 illustrates a plunger tip 2200 and frame structure 2250 capable of use with a spring-loaded injector (not shown). The plunger tip 2200 and frame structure 2250 can be coupled to an injector as previously described herein (e.g., with respect to FIGS. 17D-17F- not shown). In some embodiments, the injector is spring-loaded, such that the injector includes and/or is coupled to at least one spring element. The spring element(s) can facilitate engagement of the plunger tip 2200 and/or frame structure 2250 with an AIOL (not shown), e.g., by applying a spring force that is transmitted to an AIOL via the plunger tip 2200 and/or frame structure 2250. Configurations of spring-loaded injectors suitable for use with the embodiments disclosed herein are known to those of skill in the art. For example, in some embodiments, a spring-loaded injector is configured such that actuation of the injector or a component thereof (e.g., pushing on a plunger of the injector) compresses at least one spring element. When the user releases the injector, the spring element(s) revert towards their uncompressed state, thus causing the injector or component thereof to spring back. Accordingly, this configuration can allow an AIOL to be controllably delivered from the injector with repeated actuations (e.g., pushes). As another example, a spring-loaded injector can be configured with a screw-like mechanism, such that the injector or a component thereof is actuated by rotation (e.g., rotation of a plunger of the injector). The rotation of the injector or component thereof can compress at least one spring element. When the injector is released, the spring element(s) revert towards their uncompressed state, thus causing the injector or component thereof to spring back along a rotational trajectory. This configuration can allow an AIOL to be controllably delivered from the injector with repeated rotational actuations of the injector or component thereof.

FIG. 23 illustrates a plunger tip 2300 and frame structure 2350 with a spring element 2360. The spring element 2360 (e.g., a helical spring, resilient sleeve, or other elastic element or spring structure) can be coupled to the frame structure 2350 immediately proximal to the ring 2352 to apply a distally-directed force against the frame structure 2350. The frame structure 2350 can transmit this force to the plunger tip 2300 via contact between the ring 2352 and the body portion 2302 of the plunger tip 2300. The force applied by the spring element 2360 can improve the engagement of the plunger tip 2300 and/or frame structure 2350 with an AIOL (not shown). In some embodiments, when frictional and/or interference forces between the frame structure 2350 and an inner wall of an injector tip overcome the biasing force of the spring element 2360, the frame structure 2350 is configured to retract from the body portion 2302 of the plunger tip 2300.

In some embodiments, the spring element 2360 is used to control the force or load applied to an AIOL during delivery. For example, the plunger tip 2300 and frame structure 2350 can be coupled to an injector as previously described herein (e.g., with respect to FIGS. 17D-17F), such that actuation of the injector compresses the spring element 2360. During AIOL delivery, the user can actuate the injector until the spring element 2360 is loaded (e.g., the user feels a stop). The spring element 2360 can decompress, thereby transmitting the load to the frame structure 2350 and/or plunger tip 2300, which in turn pushes the AIOL in a distal direction. When the AIOL stops moving, the user can then actuate the injector again to re-load the spring element 2360 and push the AIOL further along the distal direction. This process can be repeated to deliver the AIOL into the eye through a series of incremental distal movements. As a result, the forces on the AIOL can be controlled (e.g., limited) by the spring element 2360, rather than by the user. This incremental delivery method is expected to improve AIOL delivery by increasing the likelihood that the AIOL is delivered in the desired orientation (e.g., without flipping or inverting), reducing the likelihood of damage to the AIOL (e.g., due to excessive forces or pressures), and/or allowing for a simpler actuation procedure (e.g., one-handed actuation). Additionally, in some embodiments, this method allows the fluid within the AIOL to pass from the portion of the AIOL within the distal portion of the injector tip to the portion of the AIOL distal to the injector tip before additional force is applied to the AIOL. As a result, the internal pressure in the portion of the AIOL compressed within the distal portion of the injector tip can be reduced. This fluid transfer and consequent expansion to the portion of the AIOL distal to the injector tip can also pull the AIOL along the distal direction, thus allowing for continued distal movement of the AIOL even after the spring element 2360 has fully decompressed.

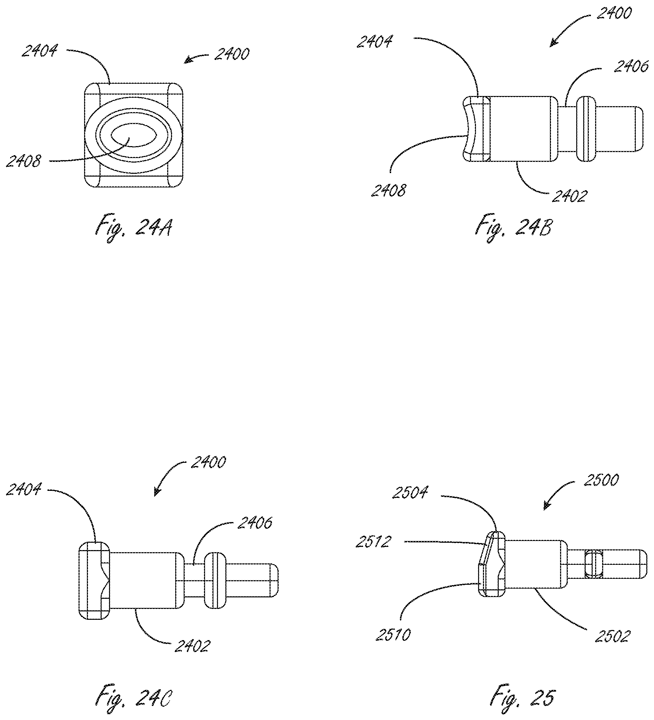

FIGS. 24A-24C illustrate a plunger tip 2400 having an end portion 2404 with a square or rectangular cross-sectional shape. The plunger tip 2400 can also include an elongated body portion 2402 and a narrowed neck portion 2406 (e.g., for coupling to a frame structure (not shown)). The geometry of the end portion 2404 can be varied as desired to facilitate engagement with an AIOL. For example, the end portion 2404 can include a concave distal face 2408 shaped to engage a corresponding surface of the AIOL.

FIG. 25 illustrates a plunger tip 2500 having a beveled end portion 2504. The plunger tip 2500 can be generally similar to the plunger tip 2400 described with respect to FIGS. 24A-24C, except that the end portion 2504 includes a flat surface 2510 and a beveled surface 2512.

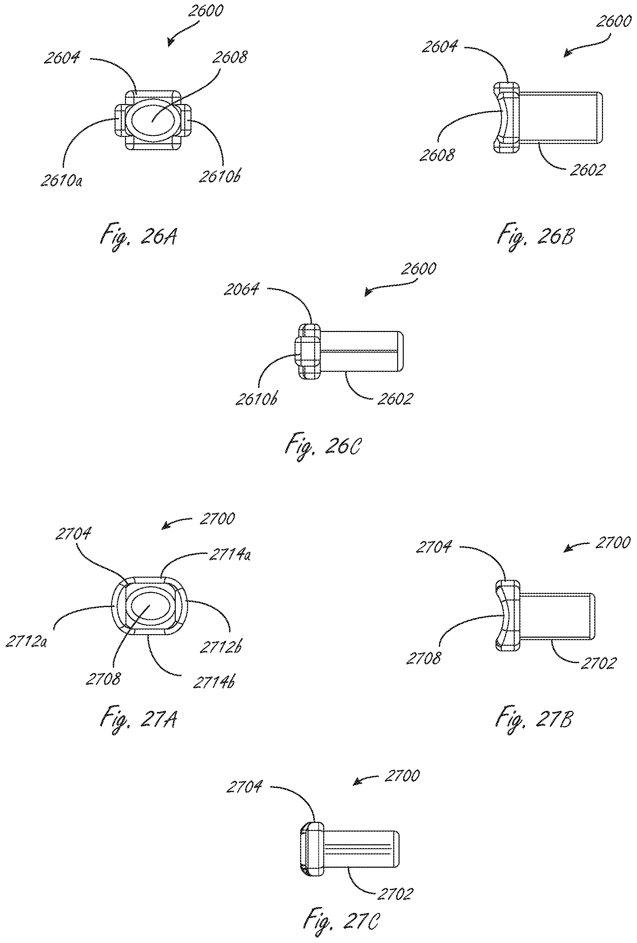

FIGS. 26A-26C illustrate a plunger tip 2600 having an end portion 2604 including a pair of protrusions 2610a and 2610b (collectively, 2610). The end portion 2604 can be connected to an elongated body portion 2602. In some embodiments, the plunger tip 2600 is configured to be used without a frame structure, such that the plunger tip 2600 does not include any narrowed neck portions. The end portion 2604 can include a concave distal face 2608, similar to the end portion 2504 described with respect to FIGS. 24A-24C. The end portion 2604 can have a square or rectangular cross-sectional shape with the protrusions 2610 being located on opposite lateral sides of the end portion 2604. The protrusions 2610 can each have a square or rectangular cross-sectional shape.

FIGS. 27A-27C illustrate a plunger tip 2700 having a rounded end portion 2704. The end portion 2704 can be connected to an elongated body portion 2702. In some embodiments, the plunger tip 2700 is configured to be used without a frame structure, such that the plunger tip 2700 does not include any narrowed neck portions. The end portion 2704 can include a concave distal surface 2708, similar to the embodiments described with respect to FIGS. 24A-24C and FIGS. 26A-26C. The end portion 2704 can have a rounded shape in which opposing lateral sides 2712a and 2712b are curved and opposing lateral sides 2714a and 2714 b are straight.

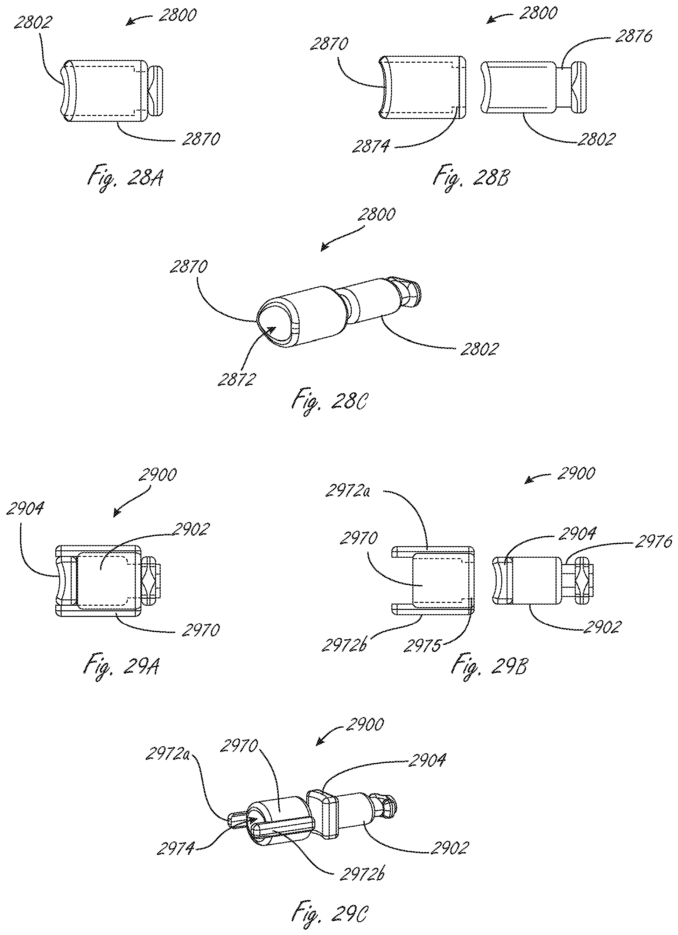

FIGS. 28A-28C illustrate a plunger tip 2800 having a sheath structure 2870. The sheath structure 2870 is positioned around an elongated body portion 2802. The body portion 2802 can be generally similar to the body portion 1802 described with respect to FIGS. 18A and 18B. The sheath structure 2870 can be an elongated hollow component having a lumen 2872 shaped to receive the body portion 2802. In some embodiments, the sheath structure 2870 includes an internal flange 2874 and/or one or more internal protrusions configured to releasably couple with an indentation or neck portion 2876 on the body portion 2802. In some embodiments, the body portion 2802 and the sheath structure 2870 each have an elliptical cross-sectional shape. The body portion 2802 and sheath structure 2870 can be arranged in a telescoping configuration to facilitate delivery of an AIOL, with the body portion 2802 serving as the inner member and the sheath structure 2870 serving as the outer member. This approach can be generally similar to the approach previously described with respect to FIGS. 17L-17S, except that the sheath structure 2870 is used instead of a frame structure. For example, during a first stage of AIOL delivery, the body portion 2802 can be located within the sheath structure 2870 so that both the body portion 2802 and the sheath structure 2870 contact and push against the AIOL. During a second, subsequent stage of AIOL delivery, the body portion 2802 can be moved distally out of the sheath structure 2870 so that body portion 2802 contacts and pushes against the AIOL without the sheath structure 2870.

FIGS. 29A-29C illustrate a plunger tip 2900 having a sheath structure 2970 with prongs 2972a-b. The plunger tip 2900 can have an elongated body portion 2902 coupled to an end portion 2904. The body portion 2902 and end portion 2904 can be generally similar to the corresponding embodiments described with respect to FIGS. 24A-24C. The sheath structure 2970 can be an elongated hollow component including a lumen 2974 shaped to receive the body portion 2902 and/or the end portion 2904. In some embodiments, the sheath structure 2970 includes an internal flange 2975 and/or one or more internal protrusions configured to releasably couple with an indentation or neck portion 2976 on the body portion 2902. A pair of prongs 2972a-b can be coupled to the exterior surface of the sheath structure 2970. The prongs 2972a-b can have a length greater than the length of the sheath structure 2970, such that the prongs 2972a-b extend past the distal end of the sheath structure 2970. In some embodiments, the sheath structure 2970 includes more than two prongs 2972 attached thereto.

The body portion 2902 and sheath structure 2970 can be arranged in a telescoping configuration to facilitate delivery of an AIOL, similar to the approach described with respect to FIGS. 28A-28C. For example, during a first stage of AIOL delivery, the body portion 2902 can be located within the sheath structure 2970, with the end portion 2904 extending distally outwards from the sheath structure 2970 and between the prongs 2972a-b. Thus, both the end portion 2904 and the prongs 2972a-b can contact and push against the AIOL. During a second, subsequent stage of AIOL delivery, the body portion 2902 can be moved distally out of the sheath structure 2970 and past the prongs 2972, so that the end portion 2904 contacts and pushes against the AIOL without the prongs 2972a-b.

As one of skill in the art will appreciate from the disclosure herein, various components of the AIOL delivery systems described above can be omitted without deviating from the scope of the present technology. Likewise, additional components not explicitly described above may be added to the AIOL delivery systems without deviating from the scope of the present technology. Accordingly, the systems described herein are not limited to those configurations expressly identified, but rather encompasses variations and alterations of the described systems.

### Conclusion

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, any of the features of the injectors described herein may be combined with any of the features of the other injectors described herein and vice versa. Moreover, although steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions associated with injectors have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A tip assembly for delivering an AIOL into a patient's eye, the tip assembly comprising:
an injector tip (1740) configured to receive the AIOL, the injector tip including a proximal portion (1742) and a distal portion (1743) configured to be inserted at least partially into the patient's eye, wherein the injector tip has a tapered shape such that the proximal portion (1742) is wider than the distal portion (1743);
a plunger assembly (1760) positionable at least partially within the injector tip (1740), the plunger assembly being movable between a first configuration and a second configuration to push the AIOL distally out of the injector tip (1740) and into the patient's eye, wherein the plunger assembly includes:
a plunger tip (1764) comprising
a widened end portion (1774) shaped to engage the AIOL and
an elongated body portion (1773) coupled to the widened end portion, and
a frame structure (1766) coupled to the plunger tip (1764) in a telescoping arrangement such that:
(a) when the plunger assembly (1760) is in the first configuration, the plunger tip (1764) is positioned at least partially within the frame structure (1766) so that the plunger tip and frame structure both engage the AIOL, and
(b) when the plunger assembly (1760) is in the second configuration, the plunger tip (1764) is positioned distally relative to the frame structure so that the plunger tip engages the AIOL without the frame structure.

2. The tip assembly of claim 1, wherein the plunger tip (1764) and the frame structure (1743) have a combined cross-sectional dimension that is narrower than the proximal portion (1742) of the injector tip and wider than the distal portion (1743) of the injector tip.

3. The tip assembly of claim 1 or 2, wherein the plunger tip (1764) is made of silicone.

4. The tip assembly of claim 1, wherein the widened end portion comprises a concave distal face.

5. The tip assembly of any one of claims 1 to 4, wherein the frame structure (1743) comprises a ring and at least one prong extending distally from the ring.

6. The tip assembly of any one of claims 1 to 5, wherein the tapered shape of the injector tip (1740) is configured to deform the AIOL from a resting configuration to a compressed configuration as the AIOL moves distally through the injector tip.

7. The tip assembly of claim 6, wherein a cross-sectional dimension of the AIOL in the resting configuration is wider than the distal portion (1743) and narrower than the proximal portion (1742).

8. The tip assembly of any one of claims 1 to 7, wherein the distal portion (1743) of the injector tip (1740) terminates in a beveled end portion.

9. The tip assembly of claim 1, wherein the distal portion of the injector tip (1740) has a cross-sectional dimension less than or equal to 3.5 mm.

10. The tip assembly of any one of claims 1 to 8, wherein the injector tip (1740) includes a low-friction coating having a coefficient of friction less than or equal to 10 N.

11. The tip assembly of any one of claims 1 to 10, further comprising an adapter (1732) configured to couple the injector tip (1740) to an injector.

12. The tip assembly of claim 11, wherein the injector comprises a plunger configured for actuation by a user, and wherein the plunger assembly is configured to couple to the plunger such that the actuation of the plunger moves the plunger assembly distally and displaces the AIOL out of the injector tip.

## Patentansprüche

1. Spitzenanordnung zur Abgabe einer AIOL in ein Auge eines Patienten, wobei die Spitzenanordnung umfasst:
eine Injektorspitze (1740), die dazu konfiguriert ist, die AIOL aufzunehmen, wobei die Injektorspitze einen proximalen Abschnitt (1742) und einen distalen Abschnitt (1743) einschließt, der dazu konfiguriert ist, zumindest zum Teil in das Auge des Patienten eingesetzt zu werden, wobei die Injektorspitze eine verjüngte Form aufweist, so dass der proximale Abschnitt (1742) breiter als der distale Abschnitt (1743) ist;
eine Kolbenanordnung (1760), die zumindest zum Teil innerhalb der Injektorspitze (1740) positionierbar ist, wobei die Kolbenanordnung zwischen einer ersten Konfiguration und einer zweiten Konfiguration bewegbar ist, um die AIOL distal aus der Injektorspitze (1740) heraus und in das Auge des Patienten zu drücken, wobei die Kolbenanordnung einschließt:
eine Kolbenspitze (1764), umfassend
einen aufgeweiteten Endabschnitt (1774), der dazu geformt ist, die AIOL in Eingriff zu nehmen, und
einen länglichen Körperabschnitt (1773), der an den aufgeweiteten Endabschnitt gekoppelt ist, und
eine Rahmenstruktur (1766), die an die Kolbenspitze (1764) in einer zusammenschiebbaren Anordnung gekoppelt ist, so dass:
(a) wenn die Kolbenanordnung (1760) in der ersten Konfiguration ist, die Kolbenspitze (1764) zumindest zum Teil innerhalb der Rahmenstruktur (1766) positioniert ist, so dass die Kolbenspitze und die Rahmenstruktur beide die AIOL in Eingriff nehmen, und
(b) wenn die Kolbenanordnung (1760) in der zweiten Konfiguration ist, die Kolbenspitze (1764) in Bezug auf die Rahmenstruktur distal positioniert ist, so dass die Kolbenspitze die AIOL ohne die Rahmenstruktur in Eingriff nimmt.

2. Spitzenanordnung nach Anspruch 1, wobei die Kolbenspitze (1764) und die Rahmenstruktur (1743) eine kombinierte Querschnittsabmessung aufweisen, die schmaler als der proximale Abschnitt (1742) der Injektorspitze und breiter als der distale Abschnitt (1743) der Injektorspitze ist.

3. Spitzenanordnung nach Anspruch 1 oder 2, wobei die Kolbenspitze (1764) aus Silikon hergestellt ist.

4. Spitzenanordnung nach Anspruch 1, wobei der aufgeweitete Endabschnitt eine konkave distale Fläche umfasst.

5. Spitzenanordnung nach einem der Ansprüche 1 bis 4, wobei die Rahmenstruktur (1743) einen Ring und mindestens eine Zacke, die sich distal von dem Ring erstreckt, umfasst.

6. Spitzenanordnung nach einem der Ansprüche 1 bis 5, wobei die verjüngte Form der Injektorspitze (1740) dazu konfiguriert ist, die AIOL aus einer Ruhekonfiguration in eine komprimierte Konfiguration zu verformen, während sich die AIOL distal durch die Injektorspitze bewegt.

7. Spitzenanordnung nach Anspruch 6, wobei eine Querschnittsabmessung der AIOL in der Ruhekonfiguration breiter als der distale Abschnitt (1743) und schmaler als der proximale Abschnitt (1742) ist.

8. Spitzenanordnung nach einem der Ansprüche 1 bis 7, wobei der distale Abschnitt (1743) der Injektorspitze (1740) in einem angeschrägten Endabschnitt endet.

9. Spitzenanordnung nach Anspruch 1, wobei der distale Abschnitt der Injektorspitze (1740) eine Querschnittsabmessung kleiner als oder gleich 3,5 mm aufweist.

10. Spitzenanordnung nach einem der Ansprüche 1 bis 8, wobei die Injektorspitze (1740) eine reibungsarme Beschichtung mit einem Reibungskoeffizienten kleiner als oder gleich 10 N einschließt.

11. Spitzenanordnung nach einem der Ansprüche 1 bis 10, ferner umfassend einen Adapter (1732), der dazu konfiguriert ist, die Injektorspitze (1740) an einen Injektor zu koppeln.

12. Spitzenanordnung nach Anspruch 11, wobei der Injektor einen Kolben umfasst, der zur Betätigung durch einen Benutzer konfiguriert ist, und wobei die Kolbenanordnung dazu konfiguriert ist, sich an den Kolben zu koppeln, so dass die Betätigung des Kolbens die Kolbenanordnung distal bewegt und die AIOL aus der Injektorspitze verschiebt.

## Revendications

1. Ensemble de pointe pour délivrer une lentille intraoculaire accommodative, AIOL, dans l'œil d'un patient, l'ensemble de pointe comprenant :
une pointe d'injecteur (1740) configurée pour recevoir l'AIOL, la pointe d'injecteur incluant une partie proximale (1742) et une partie distale (1743) configurée pour être insérée au moins partiellement dans l'œil du patient, dans lequel la pointe d'injecteur a une forme effilée de sorte que la partie proximale (1742) est plus large que la partie distale (1743) ;
un ensemble de plongeur (1760) pouvant être positionné au moins partiellement au sein de la pointe d'injecteur (1740), l'ensemble de plongeur étant capable de mouvement entre une première configuration et une deuxième configuration pour pousser l'AIOL de manière distale hors de la pointe d'injecteur (1740) et jusque dans l'œil du patient, dans lequel l'ensemble de plongeur inclut :
une pointe de plongeur (1764) comprenant
une partie d'extrémité élargie (1774) façonnée pour entrer en prise avec l'AIOL, et
une partie de corps allongée (1773) couplée à la partie d'extrémité élargie, et
une structure de cadre (1766) couplée à la pointe de plongeur (1764) dans un agencement télescopique de sorte que :
(a) lorsque l'ensemble de plongeur (1760) est dans la première configuration, la pointe de plongeur (1764) est positionnée au moins partiellement au sein de la structure de cadre (1766) de sorte que la pointe de plongeur et la structure de cadre entrent toutes deux en prise avec l'AIOL, et
(b) lorsque l'ensemble de plongeur (1760) est dans la deuxième configuration, la pointe de plongeur (1764) est positionnée de manière distale par rapport à la structure de cadre de sorte que la pointe de plongeur entre en prise avec l'AIOL sans la structure de cadre.

2. Ensemble de pointe selon la revendication 1, dans lequel la pointe de plongeur (1764) et la structure de cadre (1743) ont une dimension transversale combinée qui est plus étroite que la partie proximale (1742) de la pointe d'injecteur et plus large que la partie distale (1743) de la pointe d'injecteur.

3. Ensemble de pointe selon la revendication 1 ou 2, dans lequel la pointe de plongeur (1764) est fabriquée en silicone.

4. Ensemble de pointe selon la revendication 1, dans lequel la partie d'extrémité élargie comprend une face distale concave.

5. Ensemble de pointe de pointe selon l'une quelconque des revendications 1 à 4, dans lequel la structure de cadre (1743) comprend un anneau et au moins une dent s'étendant de manière distale depuis l'anneau.

6. Ensemble de pointe selon l'une quelconque des revendications 1 à 5, dans lequel la forme effilée de la pointe d'injecteur (1740) est configurée pour déformer l'AIOL d'une configuration de repos à une configuration comprimée lorsque l'AIOL se déplace de manière distale à travers la pointe d'injecteur.

7. Ensemble de pointe selon la revendication 6, dans lequel une dimension transversale de l'AIOL dans la configuration de repos est plus large que la partie distale (1743) et plus étroite que la partie proximale (1742).

8. Ensemble de pointe selon l'une quelconque des revendications 1 à 7, dans lequel la partie distale (1743) de la pointe d'injecteur (1740) se termine par une partie d'extrémité biseautée.

9. Ensemble de pointe selon la revendication 1, dans lequel la partie distale de la pointe d'injecteur (1740) a une dimension transversale inférieure ou égale à 3,5 mm.

10. Ensemble de pointe selon l'une quelconque des revendications 1 à 8, dans lequel la pointe d'injecteur (1740) comprend un revêtement à faible frottement ayant un coefficient de frottement inférieur ou égal à 10 N.

11. Ensemble de pointe selon l'une quelconque des revendications 1 à 10, comprenant en outre un adaptateur (1732) configuré pour coupler la pointe d'injecteur (1740) à un injecteur.

12. Ensemble de pointe selon la revendication 11, dans lequel l'injecteur comprend un plongeur configuré pour être actionné par un utilisateur, et dans lequel l'ensemble de plongeur est configuré pour se coupler au plongeur de sorte que l'actionnement du plongeur déplace l'ensemble de plongeur de manière distale et déplace l'AIOL hors de la pointe d'injecteur.
